# EUROPEAN PATENT SPECIFICATION

(11) **EP 1 278 543 B1**
(45) Date of publication and mention of the grant of the patent: **19.09.2012**
(21) Application number: 01925028.1
(22) Date of filing: 13.04.2001
(51) Int. Cl.: A61K 39/395, A61K 31/635, A61K 31/60, A61K 31/57, A61K 31/52, A61P 37/06

(54) **ANTIBODY BINDING ALPHA4BETA7 INTEGRIN AND ITS USE TO TREAT INFLAMMATORY BOWEL DISEASE**
ANTIKÖRPER GEGEN ALPHA4BETA7 INTEGRIN UND SEIN VERWENDUNG ZUR BEHANDLUNG VON DARMERKRANKUNGEN
METHODE D'ADMINISTRATION D'UN ANTICORPS

(30) Priority: 14.04.2000 US 550082; 27.12.2000 US 748960
(43) Date of publication of application: 29.01.2003
(60) Divisional application: 10010997.4
(73) Proprietor: MILLENNIUM PHARMACEUTICALS, INC., Cambridge, Massachusetts 02139 (US); Genentech, Inc., South San Francisco CA 94080-4990 (US)
(72) Inventor: BRETTMAN, Lee, R., Sudbury, MA 01776 (US); FOX, Judith, A., San Francisco, CA 94115 (US); ALLISON, David, Edward, Burlingame, CA 94010 (US)
(74) Representative: Lock, Graham James
(86) International application number: PCT/US2001/012234
(87) International publication number: WO 2001/078779

(56) References cited:
- WO-A-98/06248
- US-A- 5 834 021
- US-A- 6 015 662
- GORDON FIONA H ET AL: "Treatment of active ulcerative colitis with a recombinant humanised antibody to alpha4 integrin (Antegren(R))." GASTROENTEROLOGY, vol. 116, no. 4 PART 2, April 1999 (1999-04), page A726 XP001024675 Digestive Disease Week and the 100th Annual Meeting of the American Gastroenterological Association;Orlando, Florida, USA; May 16-19, 1999 ISSN: 0016-5085
- GORDON FIONA H ET AL: "Randomised double-blind placebo-controlled trial of recombinant humanised antibody to alpha4 integrin (Antegren(R)) in active Crohn's disease." GASTROENTEROLOGY, vol. 116, no. 4 PART 2, April 1999 (1999-04), page A726 XP002179755 Digestive Disease Week and the 100th Annual Meeting of the American Gastroenterological Association;Orlando, Florida, USA; May 16-19, 1999 ISSN: 0016-5085
- LIN KO-CHUNG ET AL: "Very late antigen 4 (VLA4) antagonists as anti-inflammatory agents." CURRENT OPINION IN CHEMICAL BIOLOGY, vol. 2, no. 4, August 1998 (1998-08), pages 453-457, XP001024679 ISSN: 1367-5931
- FEAGAN B.G. N. ENGL. J. MED. no. 352, 2008, pages 2499 - 2507
- FEAGAN B.G. CLIN. GASTROENTEROL. HEPATOL. vol. 6, 2008, pages 1370 - 1377
- KENT S.J. ET AL JMRI vol. 5, 1995, pages 535 - 540
- YEDNOCK T.A. ET AL NATURE vol. 356, 1992, pages 63 - 66
- COISNE C. ET AL NATURE vol. 185, 2009, pages 5909 - 5913

## Description

### BACKGROUND OF THE INVENTION

Integrin receptors are important for regulating both lymphocyte recirculation and recruitment to sites of inflammation (Carlos, T.M. and Harlan, J.M., Blood, 84:2068-2101 (1994)). The human α4β7 integrin has several ligands, one of which is the mucosal vascular addressin MAdCAM-1 (Berlin, C., et al., Cell 74:185-195 (1993); Erle, D.J., et al., J. Immunol. 153:517-528 (1994)) expressed on high endothelial venules in mesenteric lymph nodes and Peyer's patches (Streeter, P.R., et al., Nature 331:41-46 (1988)). As such, the α4β7 integrin acts as a homing receptor that mediates lymphocyte migration to intestinal mucosal lymphoid tissue (Schweighoffer, T., et al., J. Immunol. 151-717 -729 (1993)). In addition, the α4β7 integrin interacts with fibronectin and vascular cell adhesion molecule- (VCAM-1).

Inflammatory bowel disease (IBD), such as ulcerative colitis and Crohn's disease, for example, can be a debilitating and progressive disease involving inflammation of the gastroiniestinal tract. Affecting an estimated two million people in the United States alone, symptoms include abdominal pain, cramping, diarrhea and rectal bleeding. IBD treatments have included anti-inflammatory drugs (such as, corticosteroids and sulfasalazine), immunosuppressive drugs (such as, 6-mercaptopurine, cyclosporine and azathioprine) and surgery (such as, colectomy). Podolsky, New Engl. J. Med., 315:928-937 (1991) and Podolsky, New Engl. J. Med., 325:1008-1016 (1991). However, such therapeutic agents have not been effective in maintaining remission of IBD.

. Antibodies against human α4β7 integrin, such as murine monoclonal antibody Act-1 (mAb Act-1), interfere with α4β7 integrin binding to mucosal addressin cell adhesion molecule-1 (MAdCAM-1) present on high endothelial venules in mucosal lymph nodes. Act-1 was originally isolated by Lazarovits, A.I., et al., J. Immunol. 133:1857-1862 (1984), from mice immunized with human tetanus toxoid-specific T lymphocytes and was reported to be a mouse IgG1/x antibody. More recent analysis of the antibody by Schweighoffer, T., et al., J. Immunol. 151:717-729 (1993) demonstrated that it can bind to a subset of human memory CD4+ T lymphocytes which selectively express the α4β7 integrin. However, a serious problem with using murine antibodies for therapeutic applications in humans is that they are highly immunogenic in humans and quickly induce a human anti-murine antibody response (HAMA), which reduces the efficacy of the mouse antibody in patients and can prevent continued administration. The HAMA response results in rapid clearance of the mouse antibody, severely limiting any therapeutic benefit.

Gordon, F.H. et al., Gastroenterology, vol. 116, no. 4, page A726 XP001024675, (abstract G3151), discloses the use of Antegren™, a recombinant humanized antibody to α4 integrin with activity against α4β1 and α4β7 integrins, in patients with active ulcerative colitis (UC) at a dose of 3mg/kg.

Gordon, F.H. et al, Gastroenterology, vol. 116, no. 4, page A726, XP002179755, (abstract G3152), discloses the use of Antegren™, a recombinant humanized antibody to α4 integrin with activity against α4β1 and α4β7 integrins, in patients with active Crohns's disease (CD) at a dose of 3mg/kg.

A need exists for improved therapeutic approaches to inflammatory bowel diseases and other inflammatory disorders of mucosal tissues.

### SUMMARY OF THE INVENTION

The invention relates to the use of a humanized immunoglobulin or antigen-binding fragment thereof, for the manufacture of a medicament for treating a human having inflammatory bowel disease, wherein said medicament is for administering in a single dose or in an initial dose followed by one or more subsequent doses wherein no more than about 8 mg immunoglobulin or fragment per kg body weight are administered during a period of about one month, and
wherein said humanized immunoglobulin or antigen-binding fragment thereof comprises an antigen binding region of nonhuman origin and at least a portion of an antibody of human origin and has binding specificity for the α4β7 complex, and wherein said antigen-binding region comprises three complementarity determining regions (CDR1, CDR2 and CDR3) of a light chain variable region and three-complementarity determining regions (CDR1, CDR2 and CDR3) of a heavy chain variable region of the amino acid sequence set forth below:

| | |
|---|---|
| light chain: | CDR1 SEQ ID NO:9 |
| | CDR2 SEQ ID NO:10 |
| | CDR3 SEQ ID NO:11 |
| heavy chain: | CDR1 SEQ ID NO:12 |
| | CDR2 SEQ ID NO:13 |
| | CDR3 SEQ ID NO:14. |

The invention also relates to the use of a humanized immunoglobulin or antigen-binding fragment thereof, for the manufacture of a medicament for inhibiting relapse and/or recurrence of quiescent inflammatory bowel disease wherein said medicament is for administering in a single dose or in an initial dose and subsequent doses wherein no more than about 8 mg immunoglobulin or fragment per kg body weight are administered during a period of about 30 days and wherein said antigen-binding fragment thereof comprises an antigen-binding of nonhuman origin and at least a portion of an antibody of human origin and has binding specificity for the α4β7 complex, and wherein said antigen-binding region comprises three complementarity determining regions (CDR1, CDR2 and CDR3) of a light chain variable region and three-complementarity determining regions (CDR1, CDR2 and CDR3) of a heavy chain variable region of the amino acid sequence set forth below:

| | |
|---|---|
| light chain: | CDR1 SEQ ID NO:9 |
| | CDR2 SEQ ID NO:10 |
| | CDR3 SEQ ID NO:11 |
| heavy chain: | CDR1 SEQ ID NO:12 |
| | CDR2 SEQ ID NO:13 |
| | CDR3 SEQ ID NO:14. |

LDP-02 is a preferred antibody for administration. The immunoglobulin can be for administering in multiple doses and the interval between doses can be at least 1 day or longer. In particular embodiments, the interval between doses can be at least about 7, 14 or 21 days or about one month. In one embodiment, the amount of immunoglobulin for administering per dose can be an amount which is sufficient to achieve about 50% or greater saturation of α4β7 binding sites on circulating lymphocytes and/or about 50% or greater inhibition of α4β7 integrin expression on the surface of circulating lymphocytes for a period of at least about 10 days following administration of the dose. In another embodiment, the amount of: immunoglobulin for administering per dose can be an amount which is sufficient to achieve and maintain a serum concentration of said immunoglobulin of at least about 1 µg/mL for a period of about 10 days following administration of the dose.

The immunoglobulin can be for administering alone or together with one or more other agents to treat inflammatory bowel disease. For example, the immunoglobulin can be for administering with steroids, immunosuppressive agents, non-steroidal anti-inflammatory agents or immunomodulators. In a preferred embodiment, the immunoglobulin is for treating a human having Crohn's disease or ulcerative colitis.

### BRIEF DESCRIPTION OF THE DRAWINGS

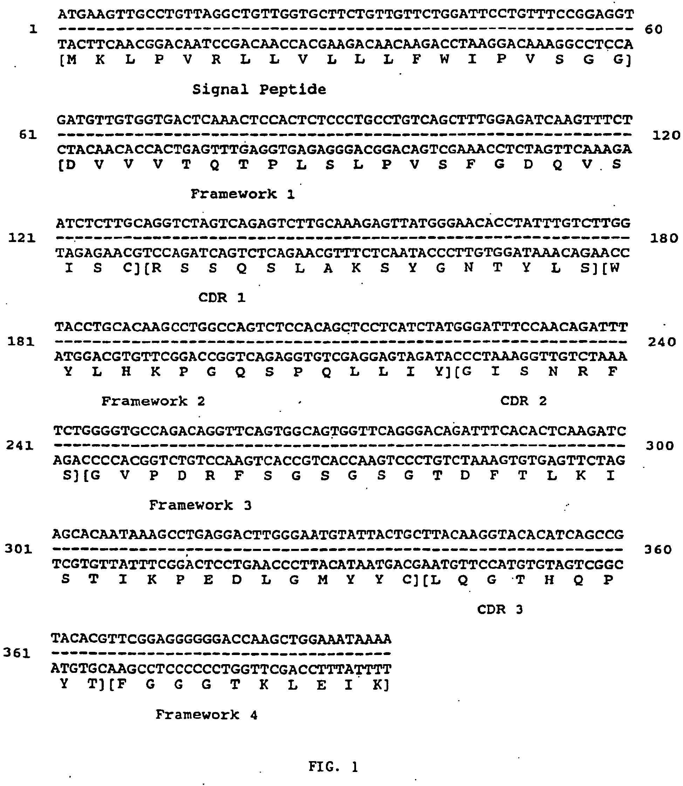

FIG. 1 is an illustration of the nucleotide sequence (SEQ ID NO:1) and deduced amino acid sequence (SEQ ID NO:2) of the mouse (*Mus musculus*) Act-1 light chain variable region joined to the mouse Act-1 light chain signal peptide sequence.

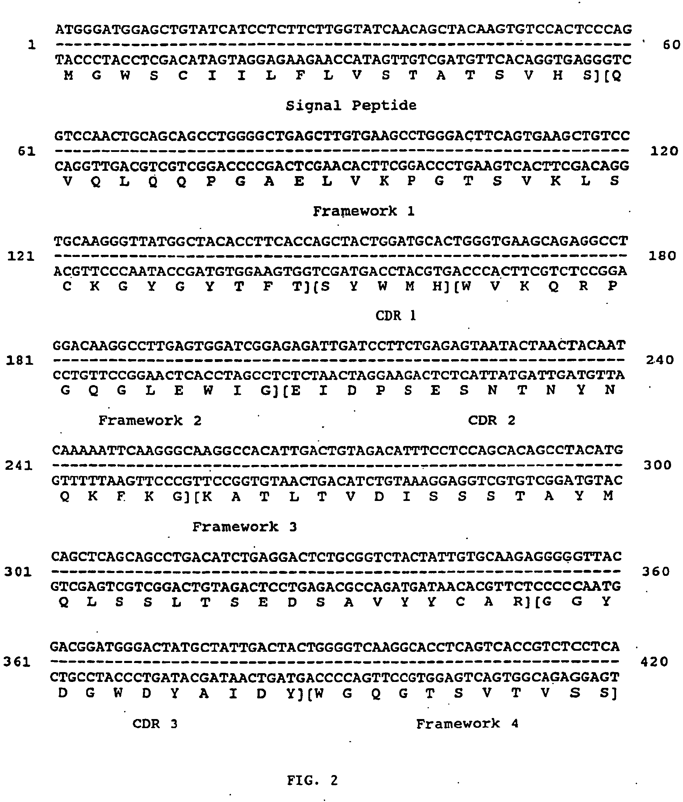

FIG. 2 is an illustration of the nucleotide sequence (SEQ ID NO:3) and amino acid sequence (SEQ ID NO:4) of the mouse Act-1 antibody heavy chain variable region. The nucleotide sequence of the variable region is joined to a nucleotide sequence which encodes a deduced mouse Act-1 heavy chain signal peptide sequence, to yield a composite sequence. (The identity of the primer which amplified the heavy chain region was deduced from the degenerate sequence, and an amino acid sequence for the signal peptide was derived from the primer, downstream sequence and sequences of other signal peptides. The signal peptide shown may not be identical to that of the Act-1 hybridoma.)

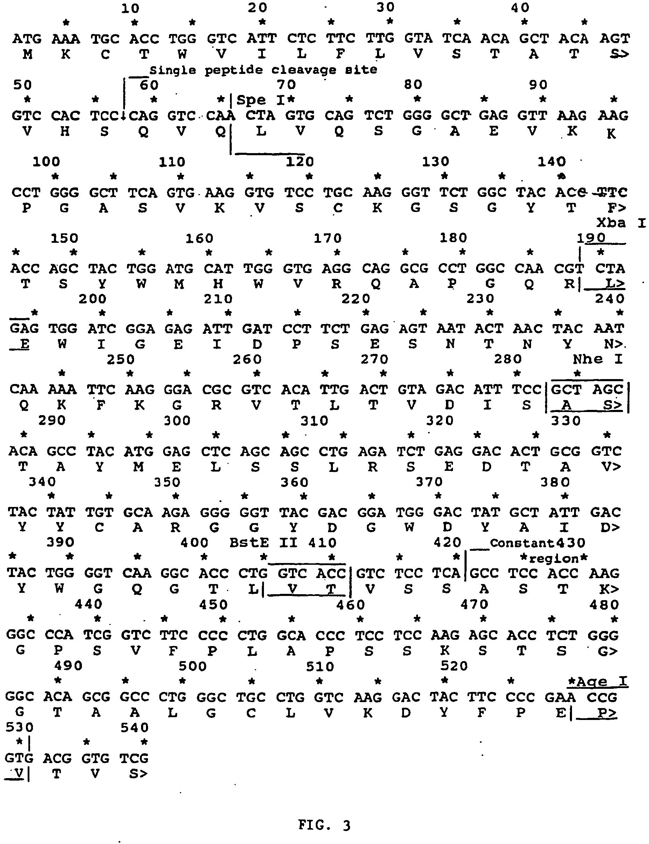

FIG. 3 is an illustration of the nucleotide sequence (SEQ ID NO:5) and amino acid sequence (SEQ ID NO:6) of a portion of the heavy chain of a humanized Act-1 antibody (LDP-02) with a heavy chain signal peptide.

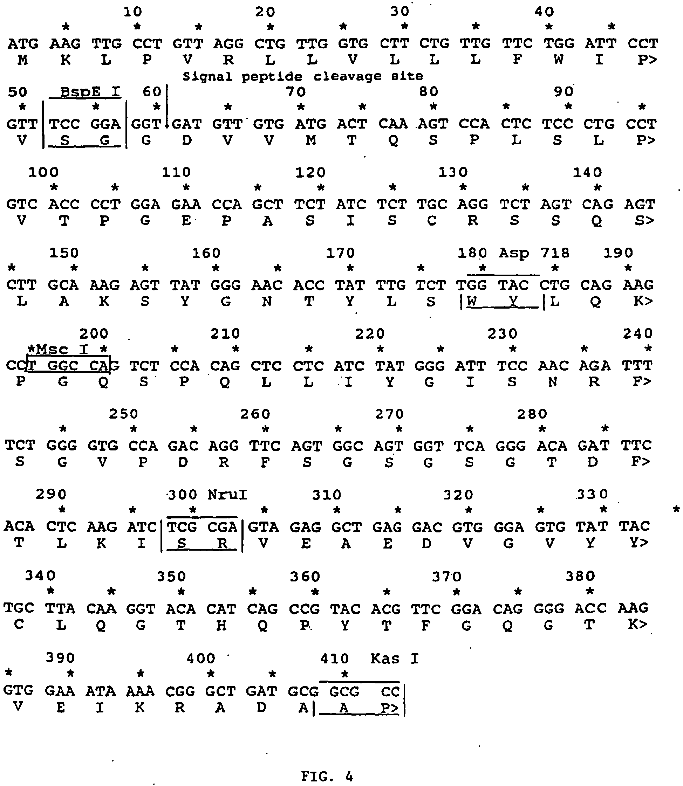

FIG. 4 is an illustration of the nucleotide sequence (SEQ ID NO:7) and amino acid sequence (SEQ ID NO:8) of a portion of the light chain of a humanized Act-1 antibody (LDP-02) with a light chain signal peptide.

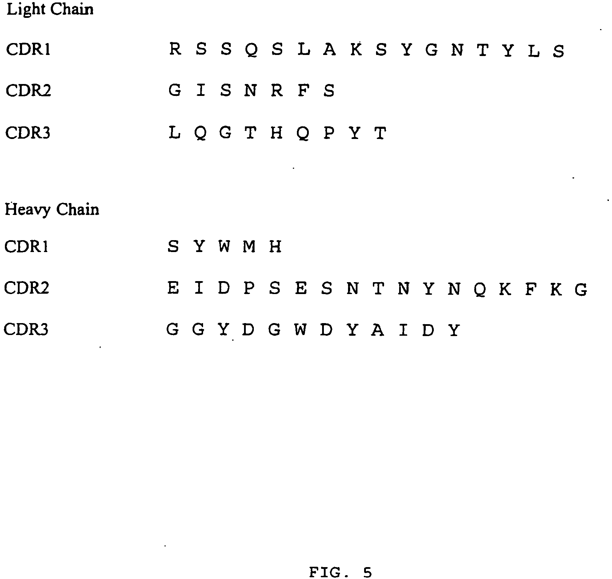

FIG. 5 is an illustration of the amino acid sequence of the light chain complementarity determining regions (CDR1, SEQ ID NO: 9; CDR2, SEQ ID NO:10; CDR3, SEQ ID NO:11) and heavy chain complementarity determining regions (CDR1, SEQ ID NO: 12; CDR2, SEQ ID NO:13; CDR3, SEQ ID NO:14) of murine antibody Act-1 and LDP-02.

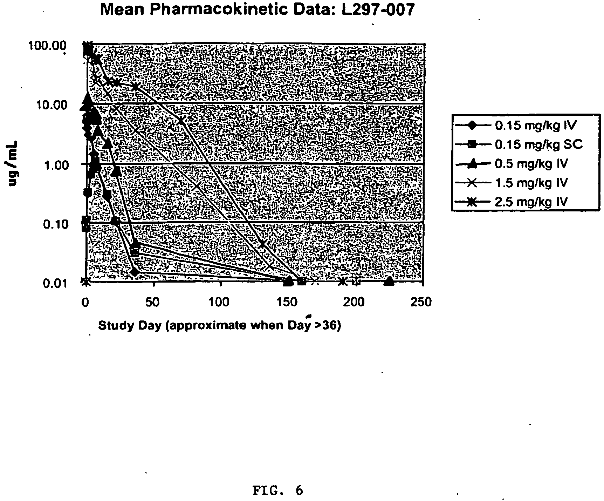

FIG. 6 is a graph showing mean serum LDP-02 levels (µg/mL) in healthy men over time following a single administration of LDP-02. Mean serum LDP-02 levels became negligible by day 36 following administration of 0.15 mg/kg by intravenous (IV)(-◆- ) or subcutaneous (SC)(- ■- ) injection and following administration of 0.5 mg/kg by intravenous injection (- ▲-). However serum LDP-02 was still measurable beyond day 36 following administration of 1.5 mg/kg (-x-) or 2.5 mg/kg (- *-) by intravenous injection.

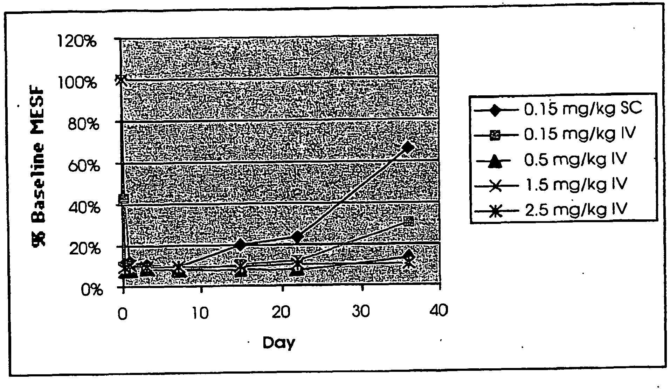

FIG. 7 is a graph showing persistent loss of α4β7 signal (detected with Act-1 mAb) following administration of LDP-02. About 90% of α4β7 signal was rapidly lost (MESF =10%) after administration of LDP-02 and persisted following administration of all LDP-02 doses. Between about day 7 and day 22, α4β7 signal started to return to baseline for the 0.15 mg/kg IV dose group (-■-) and for the 0.15 mg/kg SC dose group (-◆- ). Between day 22 and day 36, α4β7 signal started to return to baseline for the 0.5 mg/kg IV (-▲-) dose group. At the higher doses of LDP-02 studied (1.5 mg/kg (-x-) and 2.5 mg/kg (- *-)), loss of α4β7 signal persisted for longer than 36 days following single IV doses. For the 2.5 mg/kg dose group (- *-), loss of α4β7 signal persisted up to about Day 70 (data provided herein in Appendix to Study L297-007). MESF: mean equivalent soluble fluorescence.

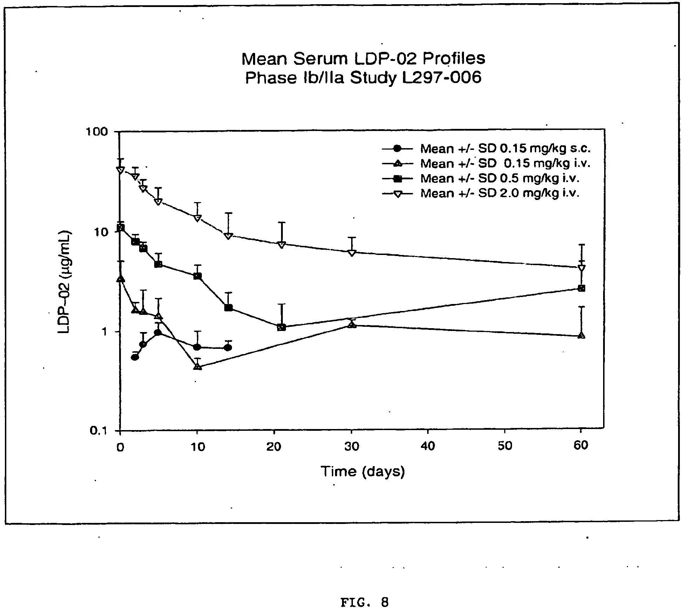

FIG. 8 is a graph showing mean serum LDP-02 levels (µg/mL) in patients with ulcerative colitis over time following a single administration of LDP-02. Mean serum LDP-02 levels rose rapidly following administration of LDP-02. The concentration of serum LDP-02 fell to below 1.0 µg/mL in patients administered LDP-02 at 0.15 mg/kg by intravenous (- ▲-) or subcutaneous (-●-) injection by 10 days following dosing. However, serum LDP-02 concentrations remained above 1.0 µg/mL for about 20 days following administration of 0.5 mg/kg by intravenous injection (- ■-). The serum concentration of LDP-02 remained above 1 µg/mL for about 60 days following administration of 2.0 mg/kg by intravenous injection (-▼-).

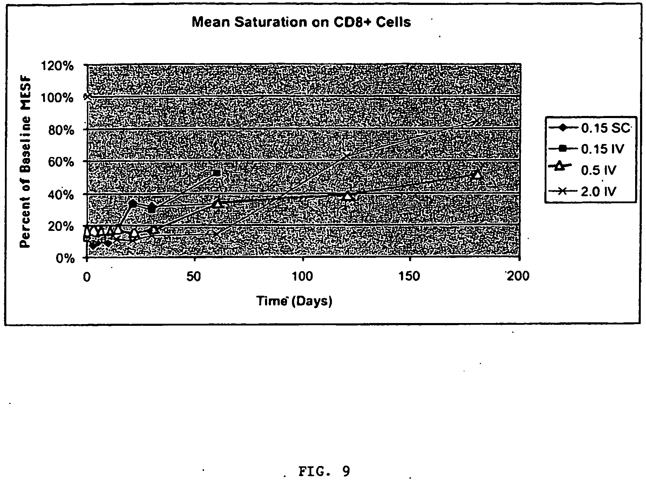

FIG. 9 is a graph showing persistent loss of α4β7 signal (detected with Act-1 mAb) following administration of LDP-02. About 90% of α4β7 signal was rapidly lost (MESF = =10%) after administration of LDP-02 and the duration of signal loss was dependent upon dose. Starting at about Day 10, α4β7 signal started to return to baseline for the group administered 0.15 mg/kg of LDP-02 by IV (- ■-) or SC (-◆-) injection. However, α4β7 signal started to return to baseline between day 30 and day 60 for the group administered 0.5 mg/kg (- Δ-) intravenously, and after day 60 for the group administered 2.0 mg/kg (-x-) intravenously (data provided herein in Appendix to Study L297-006). MESF: mean equivalent soluble fluorescence.

### DETAILED DESCRIPTION OF THE INVENTION

The humanized immunoglobulin described has binding specificity for α4β7 integrin (e.g., mammalian α4β7 (e.g., human (*Homo sapiens*) α4β7). Preferably, the humanized immunoglobulins can bind α4β7 integrin with an affinity of at least about 10⁷M⁻¹, preferably at least about 10⁸M⁻¹, and more preferably at least about 10⁹M⁻¹. The humanized immunoglobulin may include an antigen binding region of nonhuman origin which hinds α4β7 integrin and a constant region derived from a human constant region. The humanized immunoglobulin which binds α4β7 integrin may comprise a complementary determining region of nonhuman origin and a variable framework region of human origin, and if desired, a constant region of human origin. For example, the humanized immunoglobulin can comprise a heavy chain and a light chain, wherein the light chain comprises a complementarity determining region derived from an antibody of nonhuman origin which binds α4β7 integrin and a framework region derived from a light chain of human origin, and the heavy chain comprises a complementarity determining region derived from an antibody of nonhuman origin which binds α4β7 integrin and a framework region derived from a heavy chain of human origin.

Naturally occurring immunoglobulins have a common core structure in which two identical light chains (about 24 kD) and two identical heavy chains (about 55 or 70 kD) form a tctramer. The amino-terminal portion of each chain is known as the variable (V) region and can be distinguished from the more conserved constant (C) regions of the remainder of each chain. Within the variable region of the light chain is a C-terminal portion known as the J region. Within the variable region of the heavy chain, there is a D region in addition to the J region. Most of the amino acid sequence variation in immunoglobulins is confined to three separate locations in the V regions known as hypervariable regions or complementarity determining regions (CDRs) which are directly involved in antigen binding. Proceeding from the amino-terminus, these regions are designated CDR1, CDR2 and CDR3, respectively. The CDRs are held in place by more conserved framework regions (FRs). Proceeding from the amino-terminus, these regions are designated FR1, FR2, FR3, and FR4, respectively. The locations of CDR and FR regions and a numbering system have been defined by Karat *et al.* (Kabat, E.A. et al., Sequences of Proteins of Immunological Interest, Fifth Edition, U.S. Department of Health and Human Services, U.S. Government Printing Office (1991)).

Human immunoglobulins can be divided into classes and subclasses, depending on the isotype of the heavy chain. The classes include IgG, IgM, IgA, IgD and IgE, in which the heavy chains are of the gamma (γ), mu (µ), alpha (α), delta (δ) or epsilon (ε) type, respectively. Subclasses include IgG1, IgG2, IgG3, IgG4, IgA1 and IgA2, in which the heavy chains are of the γ1, γ2, γ3, γ4, α1 and α2 type, respectively. Human immunoglobulin molecules of a selected class or subclass may contain either a kappa (κ) or lambda (λ) light chain. See e.g., Cellular and Molecular Immunology, Wonsiewicz, M.J., Ed., Chapter 45, pp. 41-50, W. B. Saunders Co, Philadelphia, PA (1991); Nisonoff, A., Introduction to Molecular Immunology, 2nd Ed., Chapter 4, pp. 45-65, Sinauer Associates, Inc., Sunderland, MA (1984).

The term "immunoglobulin" as used herein includes whole antibodies and biologically functional fragments thereof. Such biologically functional fragments retain at least one antigen binding function of a corresponding full-length antibody (e.g., specificity for α4β7 of Act-1 antibody), and preferably, retain the ability to inhibit the interaction of α4β7 with one or more of its ligands (e.g., MAdCAM-1, fibronectin). In a particularly preferred embodiment, biologically functional fragments can inhibit binding of α4β7 to the mucosal addressin (MAdCAM-1). Examples of biologically functional antibody fragments which can be administered as described herein include fragments capable of binding to an α4β7 integrin, such as single chain antibodies, Fv, Fab, Fab' and F(ab')₂ fragments. Such fragments can be produced by enzymatic cleavage or by recombinant techniques. For example, papain or pepsin cleavage can generate Fab or F(ab')₂ fragments, respectively. Other proteases with the requisite substrate specificity can also be used to generate Fab, F(ab')₂ or other antigen-binding fragments. Antibodies can also be produced in a variety of truncated forms using antibody genes in which one or more stop codons have been introduced upstream of the natural stop site. For example, a chimeric gene encoding a F(ab')₂ heavy chain portion can be designed to include DNA sequences encoding the CH₁ domain and hinge region of the heavy chain.

The term "humanized immunoglobulin" as used herein refers to an immunoglobulin (antibody) comprising portions of immunoglobulins of different origin, wherein at least one portion is of human origin. For example, the humanized antibody can comprise portions derived from an immunoglobulin of nonhuman origin with the requisite specificity, such as a mouse, and from immunoglobulin sequences of human origin (e.g., chimeric immunoglobulin), joined together chemically by conventional techniques (e.g., synthetic) or prepared as a contiguous polypeptide using recombinant DNA technology (e.g., DNA encoding the protein portions of the chimeric antibody can be expressed to produce a contiguous polypeptide chain). Another example of a humanized immunoglobulin is an immunoglobulin containing one or more immunoglobulin chains comprising a CDR derived from an antibody of nonhuman origin and a framework region derived from a light and/or heavy chain of human origin (e.g., CDR-grafted antibodies with or without framework changes). Chimeric or CDR-grafted single chain antibodies are also encompassed by the term humanized immunoglobulin. See, e.g., Cabilly et al., U.S. Patent No. 4,816,567; Cabilly *et al*., European Patent No. 0,125,023 B1; Boss et al., U.S. Patent No. 4,816,397; Boss *et al.,* European Patent No. 0,120,694 B1, Neuberger, M.S. et al., WO 86/01533; Neuberger, M.S. *et al.,* European Patent No. 0,194,276 B1; Winter, U.S. Patent No. 5,225,539; Winter, European Patent No. 0,239,400 B1; Queen *et al.,* European Patent No. 0 451 216 B1; Padlan, E.A. *et al.,* European Patent. No. 0,519,596 B1. See also, Ladner et al., U.S. Patent No. 4,946,778; Huston, U.S. Patent No. 5,476,786; and Bird, R.E. et al., Science, 242: 423-426 (1988)), regarding single chain antibodies. In particular embodiments, the humanized immunoglobulin can include an immunoglobulin chain (e.g., heavy chain) having a variable region of non-human origin (e.g., murine origin) and at least a portion of a human constant region (e.g, Cγ1), and an immunoglobulin chain (e.g., light chain) where at least one CDR is of non-human origin (e.g., murine origin) and the framework regions (FR1, FR2, FR3, FR4) and, optionally, the constant region (e.g., Cκ, Cλ) are of human origin.

The antigen binding region of the humanized immunoglobulin (the nonhuman portion) can be derived from an immunoglobulin of nonhuman origin (referred to as a donor immunoglobulin) having binding specificity for α4β7 integrin. For example, a suitable antigen binding region can be derived from the murine Act-1 monoclonal antibody (Lazarovits, A.I. et al., J. Immunol., 133(4): 1857-1862 (1984)). Other sources include α4β7 integrin-specific antibodies obtained from nonhuman sources, such as rodent (e.g., mouse, rat), rabbit, pig, goat or non-human primate (e.g., monkey). Other polyclonal or monoclonal antibodies, such as antibodies which bind to the same or similar epitope as the Act-1 antibody, or LDP-02, can be made (e.g., Kohler et al., Nature, 256:495-497 (1975); Harlow et al., 1988, Antibodies: A Laboratory Manual, (Cold Spring Harbor, NY); and Current Protocols in Molecular Biology, Vol. 2 (Supplement 27, Summer '94), Ausubel et al., Eds. (John Wiley & Sons: New York, NY), Chapter 11 (1991)).

For example, antibodies can be raised against an appropriate immunogen in a suitable mammal (e.g., a mouse, rat, rabbit, sheep). Preparation of immunizing antigen, and polyclonal and monoclonal antibody production can be performed using any suitable technique. A variety of methods have been described (see e.g., Kohler et al., Nature, 256: 495-497 (1975) and Eur. J. Immunol. 6: 511-519 (1976); Milstein et al., Nature 266: 550-552 (1977*);* Koprowski et al., U.S. Patent No. 4,172,124; Harlow, E. and D. Lane, 1988, Antibodies: A Laboratory Manual, (Cold Spring Harbor Laboratory: Cold Spring Harbor, NY); Current Protocols In Molecular Biology, Vol. 2 (Supplement 27, Summer '94), Ausubel, F.M. et al., Eds., (John Wiley & Sons: New York, NY), Chapter 11, (1991)). For example, suitable immunizing agents include cells bearing a4p7, membrane fractions containing α4β7, immunogenic fragments of suitable immunogens include α4β7, a β7 peptide conjugated to a suitable carrier and the like. Antibody-producing cells (e.g., a lymphocyte) can be isolated from, for example, the lymph nodes or spleen of an immunized animal. The cells can then be fused to a suitable immortalized cell (e.g., a myeloma cell line (e.g., SP2/0, P3x63Ag8.653)), thereby forming a hybridoma. Fused cells can be isolated employing selective culturing techniques. Cells which produce antibodies with the desired specificity can be selected using a suitable assay (e.g., ELISA). Other suitable methods of producing or isolating antibodies (human antibodies, non-human antibodies) of the requisite specificity can be used, including, for example, methods which select recombinant antibody from a library (e.g., a phage display library). Transgenic animals capable of producing a repertoire of human antibodies (e.g., XenoMouse™ (Abgenix, Fremont, CA) can be produced using suitable methods (see e.g., WO 98/24893 (Abgenix), published June 11, 1998; Kucherlapate, R. and Jakobovits, A., U.S. Patent No. 5,939,598; Jakobovits et al., Proc. Natl. Acad. Sci. USA, 90: 2551-2555 (1993); Jakobovits et al., Nature, 362: 255-258 (1993)). Additional methods for production of transgenic animals capable of producing a repertoire of human antibodies have been described (e.g., Lonberg et al., U.S. Patent No. 5,545,806; Surani et al., U.S. Patent No. 5,545,807; Lonberg et al., WO97/13852).

The antigen binding region of the humanized immunoglobulin comprises CDRs of nonhuman origin derived from the light and heavy chain variable regions of immunoglobulins of nonhuman origin, such that a humanized immunoglobulin includes substantially heavy chain CDR1, CDR2 and CDR3, and light chain CDR1, CDR2 and CDR3, from one or more immunoglobulins of nonhuman origin, and the resulting humanized inununoglobulin has binding specificity for α4β7 integrin. Preferably, all three CDRs of a selected chain are substantially the same as the CDRs of the corresponding chain of a donor, and more preferably, all six CDRs of the light and heavy chains are substantially the same as the CDRs of the corresponding donor chains. The one or more CDRs of nonhuman origin have the amino acid sequences of the CDRs of murine Act-1 Ab (SEQ ID NOS: 9-14).

The portion of the humanized immunoglobulin or immunoglobulin chain which is of human origin (the human portion) can be derived from any suitable human immunoglobulin or immunoglobulin chain. For example, a human constant region or portion thereof, if present, can be derived from the κ or λ light chains, and/or the γ (e.g., γ1, γ2, γ3, γ4), µ, α (e.g., α1, α2), δ or e heavy chains of human antibodies, including allelic variants. A particular constant region (e.g., IgG1), variant or portions thereof can be selected in order to tailor effector function. For example, a mutated constant region (variant) can be incorporated into a fusion protein to minimize binding to Fc receptors and/or ability to fix complement (see e.g., Winter et al., GB 2,209,757 B; Morrison et al., WO 89/07142; Morgan et al., WO 94/29351, December 22, 1994). LDP-02 contains a heavy chain constant region (human γ1 heavy chain constant region) that was modified to reduce binding to human Fcγ receptors. The LDP-02 Fc modifications are at positions 235 and 237 (i.e., Leu²³⁵-Ala²³⁵ and Gly²³⁷-Ala²³⁷).

If present, framework regions of human origin (e.g., of the light chain variable region) are preferably derived from a human antibody variable region having sequence similarity to the analogous region (e.g., light chain variable region) of the antigen binding region donor. Other sources of framework regions for portions of human origin of a humanized immunoglobulin include human variable consensus sequences (see e.g., Kettleborough, C.A. et al., Protein Engineering 4:773-783 (1991); Carter et al., WO 94/04679, published March 3, 1994)). For example, the sequence of the antibody or variable region used to obtain the nonhuman portion can be compared to human sequences as described in Kabat, E.A., et al., Sequences of Proteins of Immunological Interest, Fifth Edition, U.S. Department of Health and Human Services, U.S. Government Printing Office (1991). In a particularly preferred embodiment, the framework regions of a humanized immunoglobulin chain are derived from a human variable region having at least about 65% overall sequence identity, and preferably at least about 70% overall sequence identity, with the variable region of the nonhuman donor antibody (e.g., mouse Act-1 antibody). A human portion can also be derived from a human antibody having at least about 65% sequence identity, and preferably at least about 70% sequence identity, within the particular portion (e.g., FR) being used, when compared to the equivalent portion (e.g., FR) of the nonhuman donor. Amino acid sequence identity can be determined using a suitable sequence alignment algorithm, such as the Lasergene system (DNASTAR, Inc., Madison, WI), using the default parameters.

In one embodiment, the humanized immunoglobulin comprises at least one of the framework regions (FR) derived from one or more chains of an antibody of human origin. Thus, the FR can include a FR1 and/or FR2 and/or FR3 and/or FR4 derived from one or more antibodies of human origin. Preferably, the human portion of a selected humanized chain includes FR1, FR2, FR3 and FR4 derived from a variable region of human origin (e.g., from a human immunoglobulin chain, from a human consensus sequence).

The immunoglobulin portions of nonhuman and human origin for use in preparing humanized antibodies can have sequences identical to immunoglobulins or immunoglobulin portions from which they are derived or to variants thereof Such variants include mutants differing by the addition, deletion, or substitution of one or more residues. As indicated above, the CDRs which are of nonhuman origin are substantially the same as in the nonhuman donor, and preferably are identical to the CDRs of the nonhuman donor. Changes in the framework region, such as those which substitute a residue of the framework region of human origin with a residue from the corresponding position of the donor, can be made. One or more mutations in the framework region can be made, including deletions, insertions and substitutions of one or more amino acids. For a selected humanized antibody or chain, suitable framework mutations can be designed. Preferably, the humanized immunoglobulins can bind α4β7 integrin with an affinity similar to or better than that of the nonhuman donor. Variants can be produced by a variety of suitable methods, including mutagenesis of nonhuman donor or acceptor human chains.

Immunoglobulins (e.g., human and/or humanized immunoglobulins) having binding specificity for human α4β7 integrin include immunoglobulins (including antigen-binding fragments) which can bind determinants (epitopes) of the α4 chain (e.g., mAb HP1/2 (Pulido, et al., J Biol Chem 266:10241-10245 (1991), murine MAb 21.6 and humanized MAb 21.6 *(*Bendig et al., U.S. Patent No. 5,840,299)) and/or the β7 chain of the α4β7 heterodimer. For example, in particular embodiments, the human or humanized immunoglobulin can specifically or selectively bind a determinant of the α4β7 complex, but not bind determinants (epitopes) on the α4 chain or the β7 chain. In one embodiment, the human or humanized immunoglobulin can have binding specificity for a combinatorial epitope on the α4β7 heterodimer. Such an immunoglobulin can bind α4β7 and not bind α4β7, for example. Antibodies which have binding specificity for the α4β7 complex include, murine Act-1 antibody, and a humanized Act-1 referred to as LDP-02 (see, WO 98/06248 by LeukoSite, Inc., published February 19, 1998 and U.S. Patent Number 7,147,852. In a preferred embodiment, the humanized immunoglobulin has at least one function characteristics of murine Act-1 antibody, such as binding function (e.g., having specificity for α4β7 integrin, having the same or similar epitopic specificity), and/or inhibitory function (e.g., the ability to inhibit α4β7-dependent adhesion *in vitro* and/or *in vivo*, such as the ability to inhibit α4β7 integrin binding to MAdCAM-1 *in vitro* and/or *in vivo,* or the ability to inhibit the binding of a cell bearing α4β7 integrin to a ligand thereof (e.g., a cell bearing MAdCAM-1)). Thus, preferred humanized immunoglobulins can have the binding specificity of the murine Act-1 antibody, the epitopic specificity of murine Act-1 antibody (e.g., can compete with murine Act-1, a chimeric Act-1 antibody, or humanized Act-1 (e.g., LDP-02) for binding to α4β7 (e.g., on a cell bearing α4β7 integrin)), and/or inhibitory function. A particularly preferred humanized Ab is LDP-02.

The binding function of a human or humanized immunoglobulin having binding specificity for α4β7 intcgrin can be detected by standard immunological methods, for example using assays which monitor formation of a complex between humanized immunoglobulin and α4β7 integrin (e.g., a membrane fraction comprising α4β7 integrin, on a cell bearing α4β7 integrin, such as a human lymphocyte (e.g., a lymphocyte of the CD4⁺α4^{hi} β1^{lo} subset), human lymphocytes cell line or recombinant host cell comprising nucleic acid encoding α4 and/or β7 which expresses α4β7 integrin). Binding and/or adhesion assays or other suitable methods can also be used in procedures for the identification and/or isolation of immunoglobulins (e.g., human and/or humanized immunoglobulins) (e.g., from a library) with the requisite specificity (e.g., an assay which monitors adhesion between a cell bearing an α4β7 integrin and a ligand thereof (e.g., a second cell expressing MAdCAM, an immobilized MAdCAM fusion protein (e.g., MAdCAM-1g chimera)), or other suitable methods.

The immunoglobulin portions of nonhuman and human origin for use in preparing humanized immunoglobulins include light chains, heavy chains and portions of light and heavy chains. These immunoglobulin portions can be obtained or derived from immunoglobulins (e.g., by *de novo* synthesis of a portion), or nucleic acids encoding an immunoglobulin or chain thereof having the desired property (e.g., binds α4β7 integrin, sequence similarity) can be produced and expressed. Humanized immunoglobulins comprising the desired portions (e.g., antigen binding region, CDR, FR, constant region) of human and nonhuman origin can be produced using synthetic and/or recombinant nucleic acids to prepare genes (e.g., cDNA) encoding the desired humanized chain. To prepare a portion of a chain, one or more stop codons can be introduced at the desired position. For example, nucleic acid (e.g., DNA) sequences coding for newly designed humanized variable regions can be constructed using PCR mutagenesis methods to alter existing DNA sequences (see e.g., Kamman, M., et al., Nucl. Acids Res. 17:5404 (1989)). PCR primers coding for the new CDRs can be hybridized to a DNA template of a previously humanized variable region which is based on the same, or a very similar, human variable region (Sato, K., et al., Cancer Research 53:851-856 (1993)). If a similar DNA sequence is not available for use as a template, a nucleic acid comprising a sequence encoding a variable region sequence can be constructed from synthetic oligonucleotides (see e.g., Kolbinger, F., Protein Engineering 8:971-980 (1993)). A sequence encoding a signal peptide can also be incorporated into the nucleic acid (e.g., on synthesis, upon insertion into a vector). If the natural signal peptide sequence is unavailable, a signal peptide sequence from another antibody can be used (see, e.g., Kettleborough, C.A., Protein Engineering 4:773-783 (1991)). Using these methods, methods described herein or other suitable methods, variants can be readily produced. In one embodiment, cloned variable regions (e.g., of LDP-02) can be mutagenized, and sequences encoding variants with the desired specificity can be selected (e.g., from a phage library; see e.g., Krebber et al., U.S. 5,514,548; Hoogenboom et al., WO 93/06213, published April 1, 1993)).

Humanized immunoglobulins as described are for treating a human having inflammatory bowel disease, such as Crohn's disease or ulcerative colitis. Treatment includes therapeutic or prophylactic treatment (e.g., maintenance therapy). The disease can be prevented or delayed (e.g., delayed onset, prolonged remission or quiescence) or the severity of disease can be reduced in whole or in part.

No more than about 8 mg of immunoglobulin per kg body weight is administered during a period of about 1 month. For example, embodiments, no more than about 7 or about 6 or about 5 or about 4 or about 3 or about 2 or about 1 mg of immunoglobulin per kg body weight is administered during a period of about 1 month. As used herein, the term "month" refers to a calendar month and encompasses periods of 28, 29, 30 and 31 days. When an antigen-binding fragment of a human or humanized immunoglobulin is to be administered, the amount which is administered during the period of about one month can be adjusted in accordance with the size of the fragment. For example, if the antigen-binding fragment is about half the size of the intact antibody by weight (e.g., measured in kDa), the amount administered during a period of about 1 month can be about 4 mg per kg body weight or less. The amount of immunoglobulin or antigen-binding fragment administered can be expressed as mg/kg body weight or using any other suitable units. For example, the amount of immunoglobulin or antigen-binding fragment administered can be expressed as moles of antigen binding sites per kg body weight. The number of moles of antigen-binding sites is dependent upon the size, quantity and valency of the immunoglobulin or fragment and can be readily determined. For example, IgG and F(ab')₂ fragments thereof are divalent and a dose which comprises 1 nanomole of IgG or F(ab')₂ fragment comprises 2 nanomoles of antigen-binding sites. The size of an antibody or antigen-binding fragment can be determined using any suitable method (e.g., gel filtration).

The human or humanized antibody or antigen-binding fragment can be administered in a single dose or in an initial dose followed by one or more subsequent doses. When multiple doses are desired, the interval between doses and the amount of immunoglobulin or antigen-binding fragment can be adjusted to achieve the desired therapeutic effect but is no more than up to about 8 mg immunoglobulin or fragment per kg body weight during a period of about 1 month. Preferably, each dose independently comprises about 0.1 to about 8 mg or about 0.1 to about 5 mg immunoglobulin or fragment per kg body weight. More preferably, each dose independently comprises about 0.1 to about 2.5 mg immunoglobulin or fragment per kg body weight. Most preferably, each dose independently comprises about 0.15, about 0.5, about 1.0, about 1.5 or about 2.0 mg immunoglobulin or fragment per kg body weight.

The interval between any two doses (e.g., initial dose and first subsequent dose, first subsequent dose and second subsequent dose) can independently vary from a few seconds or minutes to about 120 days or more. For example, the initial dose can be administered and a first subsequent dose can be administered about 1 day later. Thereafter, second and third subsequent doses can be administered at intervals of about 1 month. Generally the minimum interval between doses is a period of at least about 1 day or at least about 7 days. In particular embodiments, the minimum interval between doses is a period of at least about 14 days, or at least about 21 days or at least about 1 month (e.g., 28, 29, 30, 31 days). In additional embodiments, the interval between doses can be at least about 40, about 50, about 60, about 70, about 80, about 90, about 100, about 110 or about 120 days.

The amount of human or humanized immunoglobulin or antigen-binding fragments thereof administered in each dose can be an amount which is sufficient to produce a desired pharmacokinetic or pharmacodynamic effect. A variety of pharmacokinetic and pharmacodynamic parameters of human and/or humanized immunoglobulins or antigen-binding fragments thereof can be measured using suitable methods. For instance, pharmacodynamic parameters of antibodies and antigen-binding fragments (e.g., antigen saturation, antibody-induced inhibition of expression of antigen) can be measured using a suitable immunoassay. For example, as described herein, α4β7 signal (i.e., binding of labeled antibody to α4β7) following administration of LDP-02 was measured by flow cytometry. The results of the assay revealed that administration of LDP-02 can result in saturation of α4β7 and/or inhibition of expression of α4β7 on the surface of circulating lymphocytes.

Accordingly, each dose to be administered can comprise an amount of immunoglobulin or fragment which is sufficient to achieve a) about 50% or greater saturation of α4β7 integrin binding sites on circulating lymphocytes (e.g., CD8⁺ cells) and/or b) about 50% or greater inhibition of α4β7 integrin expression on the cell surface of circulating lymphocytes for a period of at least about 10 days following administration of the dose. In other embodiments, each dose can comprise an amount of immunoglobulin or fragment which is sufficient to achieve and maintain a) about 60% or greater, about 70% or greater, about 80% or greater or about 85% or greater saturation of α4β7 integrin binding sites on circulating lymphocytes and/or b) about 60% or greater, about 70% or greater, about 80% or greater or about 85% or greater inhibition of α4β7 integrin expression on the cell surface of circulating lymphocytes for a period of at least about 10 days following administration of the dose.

In other particular embodiments, each dose can comprise an amount of immunoglobulin or fragment which is sufficient to achieve a desired degree of saturation of α4β7 integrin binding sites on circulating lymphocytes (e.g., CD8+ cells) and/or inhibit expression of α4β7 integrin on the cell surface of circulating lymphocytes to the desired degree for a period of at least about 14 days, at least about 20 days, at least about 25 days or at least about one month following administration of the dose. In additional embodiments, each dose can comprise an amount of immunoglobulin or fragment which is sufficient to achieve a desired degree of saturation of α4β7 integrin binding sites on circulating lymphocytes (e.g., CD8+ cells) and/or inhibit expression of α4β7 integrin on the cell surface of circulating lymphocytes to the desired degree for a period of at least about 40, about 50, about 60, about 70, about 80, about 90, about 100, about 110 or about 120 days.

Suitable assays for determining the dose of antibody required to achieve a desired serum concentration or to saturate and/or inhibit expression of a target antigen can be readily designed. For example, a flow cytometry based assay can be used to measure α4β7 expression on the surface of cells isolated from a subject following administration of an immunoglobulin (e.g., human, humanized) which binds to α4β7. In one embodiment, a murine antibody which binds human α4β7 can be used. Preferably the murine antibody can bind to an epitope on α4β7 which is distinct from the epitope bound by the human or humanized immunoglobulin and the binding of the murine antibody to α4β7 is not inhibited (e.g., blocked) by the prior binding of the humanized immunoglobulin. Murine antibodies or other antibodies with these properties can be prepared and selected using the methods described herein or other suitable methods. The level of α4β7 expression on circulating lymphocytes (e.g., CD8+ cells) isolated from a human can be measured or determined using each of the antibodies (i.e., immunoglobulin to be administered, murine antibody) by flow cytometry or other suitable methods. Then, the human or humanized antibody to be administered can be administered to the human, peripheral blood can be drawn at predetermined times following the administration, and lymphocytes can be isolated (e.g., by density gradient centrifugation) for analysis. The peripheral blood lymphocytes (e.g., CD8+ cells) can be stained with each of the antibodies and the amount of α4β7 detected by each antibody can be measured or detected by flow cytometry or other suitable methods. A decrease in the amount of α4β7 integrin measured or determined using the human or humanized immunoglobulin is indicative of a) persistent integrin occupancy by the administered immunoglobulin (e.g., antigen saturation) and/or b) inhibition of α4β7 expression on the surface of the lymphocytes (e.g., down modulation of α4β7, shedding of α4β7). A decrease in the amount of α4β7 integrin measured or detected using the human or humanized immunoglobulin together with no change in the amount of α4β7 integrin measured or determined using the murine antibody is indicative of persistent occupancy of α4β7 (e:g., saturation) by the human or humanized immunoglobulin administered. A decrease in the amount of α4β7 integrin measured or detected using the human or humanized immunoglobulin together with a decrease in the amount of α4β7 integrin measured or detected using the murine antibody is indicative of inhibition of α4β7 expression on the surface of circulating lymphocytes.

Pharmacokinetic parameters, such as the serum concentration of antibody over time following administration of said antibody can be measured using an immunoassay such as an ELISA or cell-based assay. For example, as described herein, the serum concentration of a humanized anti-α4β7 immunoglobulin (LDP-02) at predetermined time points following a single administration of antibody (LDP-02) was measured using a cell-based assay. The results of the assay revealed that the serum concentration of LDP-02 can remain elevated (e.g., at or above 1 µg/mL) for a period of about 10 days or more following administration of the humanized antibody. The prolonged presence of LDP-02 in the serum can be indicative of superior efficacy as a result of persistent inhibition of α4β7 function, for example persistent inhibition of α4β7 mediated adhesion of leukocytes to MAdCAM.

Accordingly, each dose to be administered can comprise an amount of immunoglobulin or fragment which is sufficient to achieve and maintain a serum concentration of at least about 1 µg/mL for a period of at least about 10 days following administration of the dose. In particular embodiments, each dose can comprise an amount of immunoglobulin or fragment which is sufficient to achieve and maintain a serum concentration of at least about 1 µg/mL for a period of at least about 14 days, at least about 20 days, at least about 25 days or at least about one month following administration of the dose. In additional embodiments, each dose can comprise amount of immunoglobulin or fragment which is sufficient to achieve and maintain a serum concentration of at least about 1 µg/mL for a period of at least about 40, about 50, about 60, about 70, about 80, about 90, about 100, about 110 or about 120 days.

As discussed herein, antigen-binding fragments of a human or humanized immunoglobulin can be substantially smaller and, therefore, bind more antigen (α4β7) per unit of protein (µg) than intact or native immunoglobulin. Accordingly, the serum concentration of an antigen-binding fragment of a human or humanized immunoglobulin which can be indicative of superior efficacy can be lower than 1 µg/mL. Thus, when administration of an antigen-binding fragment of a human or humanized immunoglobulin is desired, the dose can comprise an amount of antigen-binding fragment which is sufficient to achieve a serum concentration which is proportionate to 1 µg/mL, for an intact immunoglobulin. For example, if the antigen-binding fragment is about half the size of the intact antibody by weight (e.g., measured in kDa), the dose can comprise an amount sufficient to achieve and maintain a serum concentration of about 0.5 µg/mL for a period of at least about 10 days. The desired serum concentration of immunoglobulin or antigen-binding fragment can be expressed as µg/mL or using any other suitable units. For example, the amount of immunoglobulin or antigen-binding fragment administered can be expressed as moles of antigen binding sites per volume of serum (e.g., M).

Human and humanized immunoglobulins can be administered for in *vivo* diagnostic applications or to modulate α4β7 integrin function in therapeutic (including prophylactic) applications. For example, human and humanized immunoglobulins can be used to detect and/or measure the level of an α4β7 integrin in a subject. For example, a humanized immunoglobulin having binding specificity for α4β7 integrin can be administered to a human and antibody-α4β7 integrin complexes which are formed can be detected using suitable methods. For example, the humanized antibody can be labeled with, for example, radionuclides (¹²⁵I, ^{III}In, technetium-99m), an epitope label (tag), an affinity label (e.g., biotin, avidin), a spin label, an enzyme, a fluorescent group or a chemiluminescent group and suitable detection methods can be used. In an application of the method, humanized immunoglobulins can be used to analyze normal versus inflamed tissues (e.g., from a human) for α4β7 integrin reactivity and/or expression (e.g. radiologically) or to detect associations between IBD or other conditions and increased expression of α4β7 (e.g., in affected tissues). The immunoglobulins described herein can be administered for assessment of the presence of α4β7 integrin in normal versus inflamed tissues, through which the presence of disease, disease progress and/or the efficacy of anti-α4β7 integrin therapy in inflammatory disease can be assessed

Human and humanized immunoglobulins (including antigen-binding fragments) can be administered to an individual to modulate (e.g., inhibit (reduce or prevent)) binding function and/or leukocyte (e.g., lymphocyte, monocyte) infiltration function of α4β7 integrin. For example, human and humanized immunoglobulins which inhibit the binding of a4p7 integrin to a ligand (i.e., one or more ligands) can be administered for the treatment of diseases associated with leukocyte (e.g., lymphocyte, monocyte) infiltration of tissues (including recruitment and/or accumulation of leukocytes in tissues), particularly of tissues which express the molecule MAdCAM. An effective amount of a human immunoglobulin or antigen-binding fragment thereof, or humanized immunoglobulin or antigen-binding fragment thereof (i.e., one or more immunoglobulins or fragments) is administered to an individual (e.g., a mammal, such as a human or other primate) in order to treat such a disease. For example, inflammatory diseases, including diseases which are associated with leukocyte infiltration of the gastrointestinal tract (including gut-associated endothelium), other mucosal tissues, or tissues expressing the molecule MAdCAM-1 (e.g., gut-associated tissues, such as venules of the lamina propria of the small and large intestine; and mammary gland (e.g., lactating mammary gland)), can be treated,

Similarly, an individual having a disease associated with leukocyte infiltration of tissues as a result of binding of leukocytes to cells (e.g., endothelial cells) expressing MAdCAM-1 can be treated.

Diseases which can be treated accordingly include inflammatory bowel disease (IBD), such as ulcerative colitis, Crohn's disease, ileitis, Celiac disease, nontropical Sprue, enteropathy associated with seronegative arthropathies, microscopic or collagenous colitis, eosinophilic gastroenteritis, or pouchitis resulting after proctocolectomy, and ileoanal anastomosis.

Pancreatitis and insulin-dependent diabetes mellitus are other diseases which can be treated. It has been reported that MAdCAM-1 is expressed by some vessels in the exocrine pancreas from NOD (nonobese diabetic) mice, as well as from BALB/c and SJL mice. Expression of MAdCAM-1 was reportedly induced on endothelium in inflamed islets of the pancreas of the NOD mouse, and MAdCAM-1 was the predominant addressin expressed by NOD islet endothelium at early stages of insulitis (Hanninen, A., et al., J. Clin. Invest., 92: 2509-2515 (1993)). Further, accumulation of lymphocytes expressing α4β7 within islets was observed, and MAdCAM-1 was implicated in the binding of lymphoma cells via α4β7 to vessels from inflamed islets (Hanninen, A., et al., J. Clin. Invest., 92: 2509-2515 (1993)).

Examples of inflammatory diseases associated with mucosal tissues which can be treated: include mastitis (mammary gland), cholecystitis, cholangitis or pericholangitis (bile duct and surrounding tissue of the liver), chronic bronchitis, chronic sinusitis, asthma, and graft versus host disease (e.g., in the gastrointestinal tract). As seen in Crohn's disease, inflammation often extends beyond the mucosal surface, accordingly chronic inflammatory diseases of the lung which result in interstitial fibrosis, such as hypersensitivity pneumonitis, collagen diseases, sarcoidosis, and other idiopathic conditions can be amenable to treatment.

Treatment can be curative, induce remission or quiescence or prevent relapse or recurrence of active disease. Treatment can be episodic or chronic (e.g., chronic treatment of active disease, to maintain quiescent disease, to induce quiescence and maintain quiescence), for example.

A humanized immunoglobulin having binding specificity for α4β7 integrin is administered to a human having inflammatory bowel disease, such as ulcerative colitis or Crohn's disease. The immunoglobulin can be administered to treat active disease and/or to maintain quiescence (i.e., inhibit relapse or recurrence).

The humanized immunoglobulin can be administered to maintain quiescence of inflammatory bowel disease which has been induced by treatment with one or more other agents (e.g., steroids (prednisone, prednisolone, adrenocorticotrophic hormone (ACTH)), cyclosporin A, FK506, antibody having binding specificity for TNFα (infliximab, CDP571), azathioprene, 6-mercaptopurine, 5-aminosalicylic acid (5-ASA) or compounds containing 5-ASA (e.g., sulfsalazine, olsalazine, balsalazide), antibiotics (e.g., metronidazole), interleukins (IL-10, IL-11), nicotine, heparin, thalidomide, lidocane) or surgery (e.g., intestinal resection).

The humanized immunoglobulin or antigen-binding fragment thereof is administered in an effective amount For therapy, an effective amount is an amount sufficient to achieve the desired therapeutic (including prophylactic) effect (such as, an amount sufficient to reduce or prevent α4β7 integrin-mediated binding to a ligand thereof and/or signalling, thereby inhibiting leukocyte adhesion and infiltration and/or associated cellular responses; an amount sufficient to induce remission or prevent relapse or recurrence of disease). The humanized immunoglobulin or antigen-binding fragment thereof can be administered in a single dose or in an initial dose followed by one or more subsequent doses as described herein. The amount of immunoglobulin or antigen-binding fragment administered in a particular dose as well as the interval between doses can depend on the characteristics of the individual, such as general health, age, sex, body weight and tolerance to drugs as well as the type and severity of disease. The skilled artisan will be able to determine appropriate dosages depending on these and other factors.

The humanized immunoglobulin can be administered to an individual (e.g., a human) alone or in conjunction with another agent (i.e., one or more additional agents). A humanized immunoglobulin can be administered before, along with or subsequent to administration of the additional agent. More than one humanized immunoglobulin which inhibits the binding of α4β7 integrin to its ligands may be administered. An antibody (e.g, human antibody, humanized antibody), such as an anti-MAdCAM-1, anti-VCAM-1, or anti-ICAM-1 antibody, which inhibits the binding of leukocytes to an endothelial ligand may be administered in addition to a humanized immunoglobulin which binds α4β7 integrin.

An additional pharmacologically active ingredient (e.g., an antiinflammatory compound, such as 5-aminosalicylic acid (5-ASA) or compounds containing 5-ASA (e.g., sulfsalazine, olsalazine, balsalazide), another non-steroidal antiinflammatory compound, or a steroidal antiinflammatory compound (e.g., prednisone, prednisolone, adrenocorticotrophic hormone (ACTH)), immunosuppressive agents (azathioprene, 6-mercaptopurine, cyclosporin A, FK506), immunomodulators (e.g., antibody having binding specificity for TNFα (infliximab, CDP571), thalidomide, interleukins (e.g., recombinant human IL-10, recombinant human IL-11)), antibiotics (e.g., metronidazole), nicotine, heparin, lidocaine) can be administered in conjunction with a humanized immunoglobulin as described.

A variety of routes of administration are possible, including, but not necessarily limited to, parenteral (e.g., intravenous, intraarterial, intramuscular, intrathecal, subcutaneous injection), oral (e.g., dietary), topical, inhalation (e.g., intrabronchial; intranasal or oral inhalation, intranasal drops), or rectal, depending on the disease or condition to be treated. Parenteral administration, particularly intravenous injection and subcutaneous injection, is preferred.

The humanized immunoglobulin or antigen-binding fragment thereof can be administered to the individual as part of a pharmaceutical or physiological composition for the treatment of inflammatory bowel disease (e.g., ulcerative colitis, Crohn's disease). Such a composition can comprise an immunoglobulin or antigen-binding fragment having binding specificity for α4β7 integrin as described herein, and a pharmaceutically or physiologically acceptable carrier. Pharmaceutical or physiological compositions for co-therapy can comprise an immunoglobulin or antigen-binding fragment having binding specificity for α4β7 integrin and one or more additional therapeutic agents. An immunoglobulin or antigen-binding fragment having binding specificity for α4β7 integrin function and an additional therapeutic agent can be components of separate compositions which can be mixed together prior to administration or administered separately. Formulation will vary according to the route of administration selected (e.g., solution, emulsion, capsule). Suitable carriers can contain inert ingredients which do not interact with the immunoglobulin or antigen-binding fragment and/or additional therapeutic agent. Standard pharmaceutical formulation techniques can be employed, such as those described in Remington's Pharmaceutical Sciences, Mack Publishing Company, Easton, PA. Suitable carriers for parenteral administration include, for example, sterile water, physiological saline, bacteriostatic saline (saline containing about 0.9% benzyl alcohol), phosphate-buffered saline, Hank's solution, Ringer's-lactate and the like. Methods for encapsulating compositions (such as in a coating of hard gelatin or cyclodextran) are known in the art (Baker, et al., "Controlled Release of Biological Active Agents", John Wiley and Sons, 1986). For inhalation, the agent can be solubilized and loaded into a suitable dispenser for administration (e.g., an atomizer, nebulizer or pressurized aerosol dispenser).

The present invention will now be illustrated by the following Examples, which are not intended to be limiting in any way.

### EXAMPLES

### Introduction

LDP-02 is a humanized IgG1 monoclonal antibody that binds α4β7 integrin, a cell surface glycoprotein present on the surface of most T and B lymphocytes. α4β7 mediates lymphocyte trafficking to gastrointestinal mucosa and gut-associated lymphoid tissue through adhesion interaction with the homing receptor MAdCAM-1. By blocking α4β7-MAdCAM-1 interactions, LDP-02 can inhibit the recruitment of leukocytes from the vasculature to the gastrointestinal mucosa, thus having a beneficial effect on the inflammatory activity in patients afflicted with inflammatory bowel disease (IBD) such as ulcerative colitis and Crohn's Disease.

This section presents information from the two LDP-02 clinical trials that have been completed. These trials include one completed Phase I study conducted in healthy subjects (Study L297-007) and one completed Phase Ib/IIa trials in patients with ulcerative colitis (UC)(Study L297-006). Table 1 describes each of the studies.

**Table 1**

| Study No. # Sites Country | Study Status | Study Design/ Population | Dosing Regimen, Dose, Route | Number of Subjects Enrolled |
|---|---|---|---|---|
| L297-007 | Completed | Phase I, randomized, double-blind, placebo-controlled, ascending single dose study. | Day 1 (single dose) | Total= 19 |
| 1 | | | 0.15 mg/kg IV | LDP-02= 14 |
| UK | | | 0.15 mg/kg SC | Placebo= 5 |
| | | | 0.5 mg/kg IV | |
| | | Healthy Male Subjects 18-50 years of age | 1.5 mg/kg IV | |
| | | | 2.5 mg/kg IV | |
| L297-006 | Completed | Phase Ib/IIa, randomized, double-blind, placebo-controlled, single rising dose, multicenter study. | Day 1 (single dose) | Total= 29 |
| 5 | | | 0.15 mg/kg SC | LDP-02= 21 |
| Canada | | | 0.15 mg/kg IV | Placebo= 8 |
| | | | 0.5 mg/kg IV | |
| | | | 2.0 mg/kg IV | |
| | | Patients with moderately severe ulcerative colitis. Prior steroid use was limited (≤20mg/day). Use of 5-ASAs was allowed. | placebo IV | |

### Example 1: Study L297-007

Study L297-007 entitled, "A Placebo-Controlled, Double-Blind, Rising Dose Study Investigating the Tolerability, Pharmacodynamics and Pharmacokinetics of LDP-02 Given by the Subcutaneous and Intravenous Routes in Healthy Male Volunteers" has been completed and final results are presented in this section.

### Study Design

Study L297-007 was a randomized, double-blind, placebo-controlled, ascending single-dose study in healthy male volunteers. Healthy male volunteers 18 to 50 years of age meeting all inclusion/exclusion criteria were enrolled in the study sequentially by study group and, within each study group, were randomly assigned to receive LDP-02 or placebo (i.e., isotonic sodium citrate buffer). To minimize risk to subjects, safety and tolerability were reviewed at each dose level prior to escalating to the next dose level. The treatment groups and numbers of subjects planned for the study are shown in Table 2.

**Table 2 Study L297-007: Study Groups**

| Group | Route of Administration * | LDP-02 | | Placebo # subjects |
|---|---|---|---|---|
| | | # subjects Dose | | |
| 1 | IV | 3 | 0.15 mg/kg | 1 |
| | SC | 3 | 0.15 mg/kg | 1 |
| 2 | IV | 3 | 0.5mg/kg) | 1 |
| 3 | IV | 3 | 1.5mg/kg | |
| 4 | IV | 3 | 2.5 mg/kg | 1 |

| | | | | |
|---|---|---|---|---|
| *SC= subcutaneous administration; IV = intravenous administration | | | | |

On study Day 1, LDP-02 or placebo was administered either SC into the thigh (Group 1 SC dosing only) or via a 30 minute constant rate IV infusion (Groups 1-4). Safety assessments included recording of adverse events, physical examinations, vital signs, clinical laboratories (i.e., hematology, blood chemistries, and urinalysis), plasma cytokine levels, and 12-lead electrocardiograms (ECGs). In addition, since this was the first clinical trial of LDP-02, continuous cardiac monitoring was carried out pre-dose through 4 hours post-dose. Blood samples were obtained to assess anti-antibody response to LDP-02, cytokine levels, serum LDP-02 concentration (pharmacokinetics), and saturation and binding site occupation of α4β7 receptors and lymphocyte subsets (pharmacodynamics). Study assessments were conducted at specified times through 36 days post-treatment. Following the results of the Day 36 pharmacokinetic and pharmacodynamic (immunological) analyses, the protocol was amended to allow additional blood draws for subjects who received LDP-02. These blood draws were used to follow LDP-02 serum levels until they became non-quantifiable (i.e., below the limit of quantification [BLQ]) and to ensure that α4β7 saturation and memory cell populations had returned to baseline (pre-dose) levels. This amendment was particularly important in the higher dose groups where the characteristics of terminal phase kinetics were not well established by Day 36.

### Study Results

### Pharmacokinetics

The assay of LDP-02 in serum was performed using a validated cell-based assay. Standards and samples were incubated with a target cell line (HUT-78) which expresses the α4β7 antigen. After washing, a fluorescently labeled polyclonal antihuman IgG1 was added. Fluorescence intensity was measured by flow cytometry and compared with the fluorescence intensity of LDP-02 standards. The effective serum concentration of LDP-02 was then defined by comparison of the sample with a standard curve generated with known concentrations of LDP-02.

Blood samples for determination of LDP-02 serum concentration were collected pre-dose, 1, 1.5, 3, 8, 12 and 24 hours after dosing, and on Days 3, 5, 7, 8, 15, 22, and 36. When it became known that LDP-02 was still detectable at Day 36, blood draws for subjects who received LDP-02 continued until levels had fallen to below the limits of quantitation of the assay. Thirteen of the 14 subjects who received LDP-02 returned for follow-up blood draws up to a maximum of 226 days post-dose.

LDP-02 concentrations over time by individual patient and mean pharmacokinetic parameters by LDP-02 dose group are presented in the Appendix to Study L297-007. Mean LDP-02 serum concentrations over time are plotted out to the last blood draw for all treatment groups in FIG. 6.

**Table 3 Study L297-007: Mean Pharmacokinetic Parameters of LDP-02 in Healthy Subjects¹**

| Pharmacokinetic Parameter | I Dose and Route of Administration of LDP-02 (number of subjects) | | | | |
|---|---|---|---|---|---|
| | 0.15 mg/kg SC (n=3) | 0.15 mg/kg IV (n=3) | 0.5 mg/kg IV (n=3) | 1.5 mg/kg IV (n=3) | 2.5 mg/kg IV (n=2) |
| Cₘₐₓ (µg/mL) | 1.112 | 7.648 | 15.760 | 118.813 | 101.749 |
| | (0.519) | (3.201) | (7.476) | (14.544) | (5.117) |
| tₘₐₓ (days) | 6.01 | 0.13 | 0.5 | 0.13 | 0.05 |
| (median & range) | (4.01 - 6.01) | (0.04 - 0.33) | (0.06-0.5) | (0.06- 0.33) | (0.04-0.06) |
| T_{1/2z} (days) | 4.33 | 4.39 | 4.02 | 14.9 | 17.1 |
| | (2.23) | (1.51) | (0.71) | (10.3) | (8.91) |
| AUC₁ | 10.4 | 19.5 | 83.6 | 660 | 1651 |
| (µg·day/mL₎ | (4.40) | (5.00) | (18.3) | (229) | (229) |
| λ_{z} (1/day) | 0.1852 | 0.1731 | 0.1763 | 0.0994 | 0.0469 |
| | (0.0735) | (0.0673) | (0.0344) | (0.1145) | (0.0244) |
| AUC | 11.4 | 20.3 | 85.1 | 755 | 1747 |
| (µg·day/mL) | (5.80) | (5.88) | (18.2) | (308) | (95.8) |
| AUC Extrapolated | 5.9 | 3.4 | 1.8 | 9.5 | 5.7 |
| % | (7.3) | (3.2) | (1.4) | (16.1) | (8.0) |
| CL* | 15.3 | 7.75 | 6.06 | 2.31 | 1.43 |
| (mLday/kg) | (6.26) | (1.93) | (1.32) | (1.19) | (0.08) |
| V_{z}^{*} | 82.5 | 46.6 | 34.3 | 54.0 | 35.9 |
| (mL/g) | (6.88) | (10.1) | (2.84) | (51.4) | (20.3) |

| | | | | | |
|---|---|---|---|---|---|
| ¹All values are mean +/- SD unless otherwise indicated. The SD appears in parenthesis. *Clearance and volume terms for the SC dose group are the apparent clearance (CL/F) and apparent volume (V₂/F). | | | | | |

Values were obtained for the mean single dose IV pharmacokinetic parameters for the 4 dose groups (C_{max'}, t_{1/2z} and AUC). Follow-up samples (i.e., those taken beyond Day 36), where the focus was on safety, allowed some further characterization of the concentration-time profiles. The difference in the t_{1/2z} values between the 2 lower dose groups (0.15 and 0.5 mg/kg) and the higher dose groups (1.5 and 2.5 mg/kg) of around 10 days could be explained in that the "true" terminal phase for the higher dose groups had not been characterized. The non-compartmental pharmacokinetics of the lower doses of LDP-02 (0.15 and 0.5 mg/kg) were well characterized and non-linear pharmacokinetics became evident as the dose was increased up to 2.5 mg/kg.

### Assessment of the Pharmacodynamic Effect of LDP-02

Fluorescece-activated cell sorting (FACS) analysis was used to measure the presence of α4β7 sites on peripheral blood lymphocytes pre- and post-LDP-02 administration. To detect α4β7 that were recognized by antibody, biotin labeled ACT-1, the murine homologue of LDP-02, was added to samples of patient blood and detected using PE-streptavidin. The standardized mean equivalent soluble fluorescence (MESF) is proportional to the number of detectable α4β7 sites.

Serum α4β7 binding over time (MESF values and percentage of baseline at each post-dose time point) are presented by individual subject and by treatment group in the Appendix to Study L297-007.

As measured by FACS analysis, mean saturation of α4β7 integrin on lymphocytes over time (i.e., to Day 36) for each treatment is presented in FIG. 7. As seen in FIG. 7, there was no detection of free α4β7 binding sites on lymphocytes for at least two weeks following administration of all LDP-02 doses. Between about day 7 and day 22, α4β7 signal started to return to baseline for the 0.15 mg/kg IV dose group and for the 0.15 mg/kg SC dose group. Between day 22 and day 36, α4β7 signal started to return to baseline for the 0.5 mg/kg IV dose group. At the higher doses of LDP-02 studied (1.5, and 2.5 mg/kg) loss of α4β7 signal persisted for longer than 36 days following single IV doses. For the 2.5 mg/kg dose group, α4β7 binding saturation continued up to Day 70 (see, data in Appendix to Study L297-007).

Follow-up blood sampling up to about Study Day 200 was done to confirm that free α4β7 binding sites on lymphocytes has returned to baseline (pre-dose) levels. The initial reappearance of free α4β7 sites appeared to occur when LDP-02 blood concentrations became non-detectable.

### Conclusions

The administration of LDP-02 at IV doses of 0.15, 0.50, 1.50, and 2.5 mg/kg and a SC dose of 0.15 mg/kg to healthy male subjects was well-tolerated.

Following administration of all LDP-02 doses there was no detection of free α4β7 binding sites on lymphocytes for approximately two weeks post-dose. Saturation of α4β7 binding sites continued for up to approximately 2 weeks post-dosing for the 0.15 mg/kg IV group and for up to approximately 3 weeks post-dosing for the 0.15 mg/kg SC and 0.5 mg/kg IV groups. Duration of effect persisted for a month or longer with the 1.5 mg/kg IV dose and continued to approximately Day 70 with 2.5 mg/kg LDP-02 IV. Follow-up samples obtained after Day 36 demonstrated that expression of free α4β7 binding sites had returned to baseline (pre-dose levels). No anti-idiotype antibodies were raised to LDP-02 indicating that it did not initiate a humoral immunogenic response. The non-compartmental pharmacokinetics of the lower doses of LDP-02 (0.15 and 0.5 mg/kg) became evident as the dose was increased up to 2.5 mg/kg.

### APPENDIX TO STUDY L297-007

LDP-02 Serum Concentration Over Time by Subject by Treatment Group. Data from individual patients are presented in Tables 4-9.

**Table 4 0.15 mg/kg LDP-02 IV**

| Subject #2 | | | Subject # 3 | | | Subject # 4 | | | Mean µg/mL (n=3) |
|---|---|---|---|---|---|---|---|---|---|
| Time (hr) | Time (day) | µg/mL | Time (hr) | Time (day) | µg/mL | Time(hr) | Time (day) | µg/mL | |
| Pre-Dose | Pre-Dose | 0.01 | Pre-Dose | Pre-Dose | 0.01 | Pre-Dose | Pre-Dose | 0.01 | 0.01 |
| 1.0 | 0.042 | 5.24 | 1.0 | 0.042 | 7.98 | 1.0 | 0.042 | 2.48 | 5.24 |
| 1.5 | 0.063 | 5.33 | 1.5 | 0.063 | 6.21 | 1.5 | 0.063 | 3.42 | 4.99 |
| 3.0 | 0.125 | 5.47 | 3.0 | 0.125 | 4.66 | 3.0 | 0.125 | 4.29 | 4.81 |
| 8.0 | 0.333 | 10.67 | 8.0 | 0.333 | 5.10 | 8.0 | 0.333 | 3.26 | 6.34 |
| 12.0 | 0.500 | 4.49 | 12.0 | 0.500 | 4.50 | 12.0 | 0.500 | 2.42 | 3.80 |
| 24.0 | 1.000 | 3.23 | 24.0 | 1.000 | 3.63 | 24.0 | 1.000 | 2.24 | 3.03 |
| 72.0 | 3.000 | 1.84 | 72.0 | 3.000 | 2.94 | 72.0 | 3.000 | 3.05 | 2.61 |
| 120.0 | 5.000 | 1.21 | 120.0 | 5.000 | 1.84 | 120.0 | 5.000 | 1.16 | 1.40 |
| 168.0 | 7.000 | 0.94 | 168.0 | 7.000 | 1.29 | 168.0 | 7.000 | 0.74 | 0.99 |
| 192.0 | 8.000 | 0.62 | 192.0 | 8.000 | 1.13 | 192.0 | 8.000 | 0.70 | 0.82 |
| 360.0 | 15.000 | 0.04 | 360.0 | 15.000 | 0.53 | 360.0 | 15.000 | 0.26 | 0.28 |
| 528.0 | 22.000 | 0.02 | 528.0 | 22.000 | 0.21 | 528.0 | 22.000 | 0.09 | 0.10 |
| 864.0 | 36.000 | 0.02 | 864.0 | 36.000 | 0.01 | 864.0 | 36.000 | 0.01 | 0.01 |
| | | | 3912.0 | 163.000 | 0.01 | 3912.0 | 163.000 | 0.01 | 0.01 |
| | | | 4920.0 | 205.000 | 0.01 | 4752.0 | 198.000 | 0.01 | 0.01 |

**Table 5 0.15 mg/kg LDP-02 SC**

| Subject # 5 | | | Subject # 6 | | | Subject # 8 | | | Mean µg/mL (n=3) |
|---|---|---|---|---|---|---|---|---|---|
| Time (hr) | Time (day) | µg/mL | Time (hr) | Time (day) | µg/mL | Time (hr) | Time (day) | µg/mL | |
| Pre-Dose | Pre-Dose | 0.01 | Pre-Dose | Pre-Dose | 0.01 | Pre-Dose | Pre-Dose | 0.01 | 0.01 |
| 1.0 | 0.042 | 0.01 | 1.0 | 0.042 | 0.01 | 1.0 | 0.042 | 0.01 | 0.01 |
| 1.5 | 0.063 | 0.01 | 1.5 | 0.063 | 0.01 | 1.5 | 0.063 | 0.01 | 0.01 |
| 3.0 | 0.125 | 0.01 | 3.0 | 0.125 | 0.01 | 3.0 | 0.125 | 0.01 | 0.01 |
| 8.0 | 0.333 | 0.06 | 8.0 | 0.333 | 0.09 | 8.0 | 0.333 | 0.09 | 0.08 |
| 12.0 | 0.500 | 0.11 | 12.0 | 0.500 | 0.12 | 12.0 | 0.500 | 0.10 | 0.11 |
| 24.0 | 1.000 | 0.12 | 24.0 | 1.000 | 0.30 | 24.0 | 1.000 | 0.55 | 0.32 |
| 72.0 | 3.000 | 0.23 | 72.0 | 3.000 | 0.81 | 72.0 | 3.000 | 0.91 | 0.65 |
| 120.0 | 5.000 | 0.54 | 120.0 | 5.000 | 0.93 | 120.0 | 5.000 | 1.13 | 0.86 |
| 168.0 | 7.000 | 0.71 | 168.0 | 7.000 | 0.88 | 168.0 | 7.000 | 1.70 | 1.10 |
| 192.0 | 8.000 | 0.62 | 192.0 | 8.000 | 0.81 | 192.0 | 8.000 | 1.05 | 0.83 |
| 360.0 | 15.000 | 0.28 | 360.0 | 15.000 | 0.08 | 360.0 | 15.000 | 0.53 | 0.30 |
| 528.0 | 22.000 | 0.02 | 528.0 | 22.000 | 0.03 | 528.0 | 22.000 | 0.26 | 0.11 |
| 864.0 | 36.000 | 0.04 | 864.0 | 36.000 | 0.04 | 864.0 | 36.000 | 0.01 | 0.03 |
| 3912.0 | 163.000 | 0.01 | 3912.0 | 163.000 | 0.01 | 3912.0 | 163.000 | 0.01 | 0.01 |
| 5088.0 | 212.000 | 0.01 | 5088.0 | 212.000 | 0.01 | 5088.0 | 212.000 | 0.01 | 0.01 |

**Table 6 0.5 mg/kg LDP-02 IV**

| Subject # 9 | | | Subject # 10 | | | Subject # 12 | | | Mean µg/mL (n=3) |
|---|---|---|---|---|---|---|---|---|---|
| Time (hr) | Time (day) | µg/mL | Time (hr) | Time (day) | µg/mL | Time (hr) | Time (day) | µg/mL | |
| Pre-Dose | Pre-Dose | 0.01 | Pre-Dose | Pre-Dose | 0.01 | Pre-Dose | Pre-Dose | 0.01 | 0.01 |
| 1.0 | 0.042 | 9.06 | 1.0 | 0.042 | 10.74 | 1.0 | 0.042 | 10.93 | 10.24 |
| 1.5 | 0.063 | 24.39 | 1.5 | 0.063 | 6.62 | 1.5 | 0.063 | 8.17 | 13.06 |
| 3.0 | 0.125 | 16.37 | 3.0 | 0.125 | 10.14 | 3.0 | 0.125 | 9.94 | 12.15 |
| 8.0 | 0.333 | 15.04 | 8.0 | 0.333 | 9.30 | 8.0 | 0.333 | 9.35 | 11.23 |
| 12.0 | 0.500 | 10.64 | 12.0 | 0.500 | 11.70 | 12.0 | 0.500 | 11.19 | 11.18 |
| 24.0 | 1.000 | 9.17 | 24.0 | 1.000 | 9.00 | 24.0 | 1.000 | 8.52 | 8.90 |
| 72.0 | 3.000 | 5.34 | 72.0 | 3.000 | 7.55 | 72.0 | 3.000 | 7.60 | 6.83 |
| 120.0 | 5.000 | 10.25 | 120.0 | 5.000 | 2.43 | 120.0 | 5.000 | 8.58 | 7.09 |
| 168.0 | 7.000 | 5.74 | 168.0 | 7.000 | 6.59 | 168.0 | 7.000 | 4.93 | 5.75 |
| 192.0 | 8.000 | 3.79 | 192.0 | 8.000 | 2.48 | 192.0 | 8.000 | 4.32 | 3.53 |
| 360.0 | 15.000 | 1.70 | 360.0 | 15.000 | 2.21 | 360.0 | 15.000 | 2.49 | 2.13 |
| 528.0 | 22.000 | 0.41 | 528.0 | 22.000 | 0.12 | 528.0 | 22.000 | 1.65 | 0.73 |
| 864.0 | 36.000 | 0.01 | 864.0 | 36.000 | 0.01 | 864.0 | 36.000 | 0.11 | 0.04 |
| 3576.0 | 149.00 | 0.01 | 3912.0 | 163.000 | 0.01 | 3576.0 | 149.000 | 0.01 | 0.01 |
| | | | 5424.0 | 226.000 | 0.01 | | | | 0.01 |

**Table 7 1.5 mg/kg LDP-02 IV**

| Subject # 13 | | | Subject #15 | | | Subject # 16 | | | Mean µg/mL (n=3) |
|---|---|---|---|---|---|---|---|---|---|
| Time (hr) | Time (day) | µg/mL | Time (hr) | Time (day) | µg/mL | Time (hr) | Time (day) | µg/mL | |
| Pre-Dose | Pre-Dose | 0.01 | Pre-Dose | Pre-Dose | 0.01 | Pre-Dose | Pre-Dose | 0.01 | 0.01 |
| 1.0 | 0.042 | 87.62 | 1.0 | 0.042 | 58.06 | 1.0 | 0.042 | 103.10 | 82.93 |
| 1.5 | 0.063 | 63.67 | 1.5 | 0.063 | 134.97 | 1.5 | 0.063 | 86.05 | 94.90 |
| 3.0 | 0.125 | 92.78 | 3.0 | 0.125 | 63.78 | 3.0 | 0.125 | 106.78 | 87.78 |
| 8.0 | 0.333 | 114.69 | 8.0 | 0.333 | 64.12 | 8.0 | 0.333 | 84.42 | 87.74 |
| 12.0 | 0.500 | 73.02 | 12.0 | 0.500 | 43.76 | 12.0 | 0.500 | 44.09 | 53.62 |
| 24.0 | 1.000 | 99.61 | 24.0 | 1.000 | 77.77 | 24.0 | 1.000 | 71.80 | 83.06 |
| 72.0 | 3.000 | 102.88 | 72.0 | 3.000 | 38.82 | 72.0 | 3.000 | 67.61 | 69.77 |
| 120.0 | 5.000 | 42.46 | 120.0 | 5.000 | 25.26 | 120.0 | 5.000 | 23.95 | 30.56 |
| 168.0 | 7.000 | 26.10 | 168.0 | 7.000 | 18.42 | 168.0 | 7.000 | 23.85 | 22.79 |
| 192.0 | 8.000 | 46.47 | 192.0 | 8.000 | 11.90 | 192.0 | 8.000 | 19.85 | 26.07 |
| 360.0 | 15.000 | 19.83 | 360.0 | 15.000 | 5.80 | 360.0 | 15.000 | 19.54 | 15.06 |
| 528.0 | 22.000 | 10.93 | 528.0 | 22.000 | 0.11 | 528.0 | 22.000 | 13.89 | 8.31 |
| 864.0 | 36.000 | 0.19 | 864.0 | 36.000 | 0.69 | 864.0 | 36.000 | 9.49 | 3.46 |
| 1968.0 | 82.000 | 0.48 | 1968.0 | 163.000 | 0.30 | | | | 0.39 |
| 3264.0 | 136.000 | 0.01 | 3264.0 | 212.000 | 0.03 | | | | 0.02 |
| 4272.0 | 178.000 | 0.01 | 4824.0 | 201.000 | 0.01 | 3960.0 | 165.000 | 0.01 | 0.01 |

**Table 8 2.5 mg/kg LDP-02 IV**

| Subject # 18 | | | Subject # 19 | | | Mean µg/mL (n=2) |
|---|---|---|---|---|---|---|
| Time(hr) | Time (day) | µg/mL | Time (hr) | Time (day) | µg/mL | |
| Pre-Dose | Pre-Dose | 0.01 | Pre-Dose | Pre-Dose | 0.01 | 0.01 |
| 1.0 | 0.042 | 105.37 | 1.0 | 0.042 | 84.06 | 94.72 |
| 1.5 | 0.063 | 71.27 | 1.5 | 0.063 | 98.13 | 84.70 |
| 3.0 | 0.125 | 73.49 | 3.0 | 0.125 | 81.59 | 77.54 |
| 8.0 | 0.333 | 84.00 | 8.0 | 0.333 | 80.17 | 82.09 |
| 12.0 | 0.500 | 103.81 | 12.0 | 0.500 | 85.53 | 94.67 |
| 24.0 | 1.000 | 68.79 | 24.0 | 1.000 | 85.52 | 77.15 |
| 72.0 | 3.000 | 63.30 | 72.0 | 3.000 | 69.49 | 66.40 |
| 120.0 | 5.000 | 53.33 | 120.0 | 5.000 | 59.11 | 56.22 |
| 168.0 | 7.000 | 50.72 | 168.0 | 7.000 | 54.63 | 52.67 |
| 192.0 | 8.000 | 43.47 | 192.0 | 8.000 | 67.32 | 55.40 |
| 360.0 | 15.000 | 22.82 | 360.0 | 15.000 | 23.85 | 23.34 |
| 528.0 | 22.000 | 22.45 | 528.0 | 22.000 | 21.92 | 22.19 |
| 864.0 | 36.000 | 17.42 | 864.0 | 36.000 | 20.63 | 19.03 |
| 1680.0 | 70.000 | 5.48 | 1656.0 | 69.000 | 4.63 | 5.06 |
| 3312.0 | 138.000 | 0.01 | 2976.0 | 124.000 | 0.08 | 0.04 |
| 3984.0 | 166.000 | 0.01 | 3648.0 | 152.000 | 0.01 | 0.01 |
| | | | 4536.0 | 189.000 | 0.01 | 0.01 |

**Table 9 placebo group**

| Time | Time | Subject # | Subject # | Subject # | Subject # | Subject # |
|---|---|---|---|---|---|---|
| (hr) | (day) | 1 | 7 | 11 | 14 | 17 |
| Pre-Dose | Pre-Dose | Its | Its | Its | Its | Its |
| 1.0 | 0.042 | Its | Its | Its | Its | Its |
| 1.5 | 0.063 | Its | Its | Its | Its | Its |
| 3.0 | 0.125 | Its | Its | Its | Its | Its |
| 8.0 | 0.333 | Its | Its | Its | Its | Its |
| 12.0 | 0.500 | Its | Its | Its | Its | Its |
| 24.0 | 1.000 | Its | Its | Its | Its | Its |
| 72.0 | 3.000 | Its | Its | Its | Its | Its |
| 120.0 | 5.000 | Its | Its | Its | Its | Its |
| 168.0 | 7.000 | Its | Its | Its | Its | Its |
| 192.0 | 8.000 | Its | Its | Its | Its | Its |
| 360.0 | 15.000 | Its | Its | Its | Its | Its |
| 528.0 | 22.000 | Its | Its | Its | Its | Its |
| 864.0 | 36.000 | Its | Its | Its | Its | Its |

| | | | | | | |
|---|---|---|---|---|---|---|
| Its = below the limit of detection | | | | | | |

Study L297-007: Mean Pharmacokinetic Parameters by Treatment Group Data from individual patients are presented in Tables 10-14.

**Table 10 0.15 mg/kg LDP-02 IV**

| Subject | Cₘₐₓ | tₘₐₓ | AUCₜ | λ_{z} | t_{½z} | AUC | AUCₑₓₜ | V_{z} | CL |
|---|---|---|---|---|---|---|---|---|---|
| | (µg/ml) | (days) | (µg·day/ml) | (1/day) | (days) | (µg·day/ml) | (%) | (ml/kg) | (ml/day/kg) |
| 2 | 10.667 | 0.33 | 16.4 | 0.2486 | 2.79 | 16.5 | 0.3 | 36.7 | 9.11 |
| 3 | 7.984 | 0.04 | 25.3 | 0.1196 | 5.79 | 27.1 | 6.7 | 46.3 | 5.53 |
| 4 | 4.292 | 0.13 | 16.9 | 0.1510 | 4.59 | 17.5 | 3.3 | 56.9 | 8.60 |
| Mean | 7.648 | 0.13* | 19.5 | 0.1731 | 4.39 | 20.3 | 3.4 | 46.6 | 7.75 |
| SD | 3.201 | | 5.00 | 0.0673 | 1.51 | 5.88 | 3.2 | 10.1 | 1.93 |

| | | | | | | | | | |
|---|---|---|---|---|---|---|---|---|---|
| *Median value Cₘₐₓ = maximum concentration tₘₐₓ = time to maximum concentration λ_{z} = a measure of elimination t_{1/2z} = terminal half-live AUCₜ = AUCₐₙ = area under the curve using all time points AUC = AUCₑₓₜ = area under curve extrapolated AUC ext (%) = % of area under curve attributed to extrapolation extrapolation V_{z} = apparent volume of distribution CL = Clearance | | | | | | | | | |

**Table 11 0.15 mg/kg LDP-02 SC**

| Subject | Cₘₐₓ | tₘₐₓ | AUCₜ | λ_{z} | t_{½z} | AUC | AUCₑₓₜ | V_{z} | CL |
|---|---|---|---|---|---|---|---|---|---|
| | (µg/ml) | (days) | (µg·day/ml) | (1/day) | (days) | (µg·day/ml) | (%) | (ml/kg) | (ml/day/kg) |
| 5 | 0.711 | 6.01 | 7.18 | 0.2298 | 3.02 | 7.32 | 2.0 | 89.1 | 20.5 |
| 6 | 0.927 | 4.01 | 8.71 | 0.2253 | 3.08 | 8.83 | 1.4 | 75.4 | 17.0 |
| 8 | 1.699 | 6.01 | 15.4 | 0.1003 | 6.91 | 18.0 | 14.3 | 82.9 | 8.32 |
| Mean | 1.112 | 6.01* | 10.4 | 0.1852 | 4.33 | 11.4 | 5.9 | 82.5 | 15.3 |
| SD | 0.519 | | 4.40 | 0.0735 | 2.23 | 5.80 | 7.3 | 6.88 | 6.26 |

| | | | | | | | | | |
|---|---|---|---|---|---|---|---|---|---|
| *Median value | | | | | | | | | |

**Table 12 0.5 mg/kg LDP-02 IV**

| Subject | Cₘₐₓ | tₘₐₓ | AUCₜ | λ_{z} | t_{1/2z} | AUC | AUCₑₓₜ | V_{z} | CL |
|---|---|---|---|---|---|---|---|---|---|
| | (µg/ml) | (days) | (µg·day/ml) | (1/day) | (days) | (µg·day/ml) | (%) | (ml/kg) | (ml/day/kg) |
| 9 | 24.388 | 0.06 | 82.2 | 0.1586 | 4.37 | 85.1 | 3.4 | 37.0 | 5.87 |
| 10 | 11.699 | 0.50 | 66.1 | 0.2159 | 3.21 | 67.0 | 1.3 | 34.6 | 7.47 |
| 12 | 11.194 | 0.50 | 102.5 | 0.1543 | 4.49 | 103 | 0.8 | 31.4 | 4.84 |
| Mean | 15.760 | 0.50* | 83.6 | 0.1763 | 4.02 | 85.1 | 1.8 | 34.3 | 6.06 |
| SD | 7.476 | | 18.3 | 0.0344 | 0.71 | 18.2 | 1.4 | 2.84 | 1.32 |

| | | | | | | | | | |
|---|---|---|---|---|---|---|---|---|---|
| *Median value | | | | | | | | | |

**Table 13 1.5 mg/kg LDP-02 IV**

| Subject | Cₘₐₓ | tₘₐₓ | AUCₜ | λ_{z} | t_{1/2z} | AUC | AUCₑₓₜ | v_{z} | CL |
|---|---|---|---|---|---|---|---|---|---|
| | (µg/ml) | (days) | (µg·day/ml) | (1/day) | (days) | (µg·day/ml) | (%) | (ml/kg) | (ml/day/kg) |
| 13 | 114.686 | 0.33 | 854 | 0.2316 | 2.99 | 855 | 0.1 | 7.58 | 1.75 |
| 15 | 134.975 | 0.06 | 408 | 0.0336 | 20.6 | 409 | 0.2 | 109 | 3.67 |
| 16 | 106.779 | 0.13 | 719 | 0.0331 | 20.9 | 1000 | 28.1 | 45.3 | 1.50 |
| Mean | 118.813 | 0.13* | 660 | 0.0994 | 14.9 | 755 | 9.5 | 54.0 | 2.31 |
| SD | 14.544 | | 229 | 0.1145 | 10.3 | 308 | 16.1 | 51.4 | 1.19 |

| | | | | | | | | | |
|---|---|---|---|---|---|---|---|---|---|
| *Median value | | | | | | | | | |

**Table 14 2.5 mg/kg LDP-02 IV**

| Subject | Cₘₐₓ | tₘₐₓ | AUCₜ | λ_{z} | t_{1/2z} | AUC | AUCₑₓₜ | V_{z} | CL |
|---|---|---|---|---|---|---|---|---|---|
| | (µg/ml) | (days) | (µg·day/ml) | (1/day) | (days) | (µg·day/ml) | (%) | (ml/kg) | (ml/day/kg) |
| 18 | 105.367 | 0.04 | 1489 | 0.0296 | 23.4 | 1680 | 11.3 | 50.2 | 1.49 |
| 19 | 98.131 | 0.06 | 1814 | 0.0642 | 10.8 | 1815 | 0.1 | 21.5 | 1.38 |
| Mean | 101.749 | 10.05* | 1651 | 0.0469 | 17.1 | 1747 | 5.7 | 35.9 | 1.43 |
| SD | 5.117 | | 229 | 0.0244 | 8.91 | 95.8 | 8.0 | 20.3 | 0.08 |

| | | | | | | | | | |
|---|---|---|---|---|---|---|---|---|---|
| *Median value | | | | | | | | | |

L297-007: Serum α4λ7 Binding Over Time by Subject by Treatment Group. Data from individual patients are presented in Tables 15-20. For each subject the time of blood sampling, MESF of the sample and % of baseline (pre-dose) MESF is presented.

**Table 15 0.15 mg/kg LDP-02 IV**

| Subject # 2 | | | Subject # 3 | | | Subject # 4 | | | Mean | |
|---|---|---|---|---|---|---|---|---|---|---|
| Pre-Dose | 5689 | 100% | Pre-Dose | 5424 | 100% | Pre-Dose | 4177 | 100% | 5097 | 100% |
| 3 hr | 605 | 11% | 3 hr | 591 | 11% | 3 hr | 588 | 14% | 595 | 12% |
| 24 hrs | 589 | 10% | 24 hrs | 600 | 11% | 24 hrs | 631 | 15% | 607 | 12% |
| Day 3 | 501 | 9% | Day 3 | 496 | 9% | Day 3 | 548 | 13% | 515 | 10% |
| Day 7 | 474 | 8% | Day 7 | 473 | 9% | Day 7 | 512 | 12% | 487 | 10% |
| Day 15 | 1819 | 32% | Day 15 | 578 | 11% | Day 15 | 599 | 14% | 999 | 20% |
| Day 22 | 2426 | 43% | Day 22 | 558 | 10% | Day 22 | 609 | 15% | 1198 | 23% |
| 36 | 3028 | | Day 36 | 3570 | 66% | Day 36 | 3469 | 83% | 3356 | 66% |
| | | | Day 163 | 6934 | 128% | Day 163 | 6837 | 164% | 6885 | 135% |
| | | | Day 205 | 4675 | 86% | Day 205 | 6755 | 162% | 5715 | 112% |

**Table 16 0:15 mg/kg LDP-02 SC**

| Subject # 5 | | | Subject # 6 | | | Subject # 8 | | | Mean | |
|---|---|---|---|---|---|---|---|---|---|---|
| Pre-Dose | 6043 | 00% | Pre-Dose | 6779 | 100% | Pre-Dose | 5857 | 100% | 6226 | 100% |
| 3 hr | 1797 | 30% | 3 hr | 4727 | 70% | 3 hr | 1514 | 26% | 2679 | 43% |
| 24 hrs | 637 | 11% | 24 hrs | 588 | 9% | 24 hrs | 616 | 11% | 614 | 10% |
| Day 3 | 529 | 9% | Day 3 | 520 | 8% | Day 3 | 527 | 9% | 525 | 8% |
| Day 7 | 486 | 8% | Day 7 | 474 | 7% | Day 7 | 485 | 8% | 482 | 8% |
| Day 15 | 598 | 10% | Day 15 | 642 | 9% | Day 15 | 635 | 11% | 625 | 10% |
| Day 22 | 759 | 13% | Day 22 | 934 | 14% | Day 22 | 579 | 10% | 757 | 12% |
| Day 36 | 1455 | 24% | Day 36 | 1452 | 21% | Day 36 | 2799 | 48% | 1902 | 31% |
| Day 163 | 2743 | 45% | Day 163 | 1989 | 29% | Day 163 | 4621 | 79% | 3118 | 50% |
| Day 212 | 4201 | 170% | Day 212 | 2601 | 38% | Day 212 | 4832 | 82% | 3878 | 62% |

**Table 17 0.5 mg/kg LDP-02 IV**

| Subject # 9 | | | Subject # 10 | | | Subject # 12 | | | Mean | |
|---|---|---|---|---|---|---|---|---|---|---|
| Pre-Dose | 5519 | (100% | Pre-Dose | 5966 | 100% | Pre-Dose | 8550 | 100% | 6678 | 100% |
| 24 hrs | 533 | 10% | 24 hrs | 548 | 9% | 24 hrs | 539 | 6% | 540 | 8% |
| 24 hrs | 542 | 10% | 24 hrs | 554 | 9% | 24 hrs | 527 | 6% | 541 | 8% |
| Day 3 | 565 | 10% | Day 3 | 574 | 10% | Day 3 | 539 | 6% | 560 | 8% |
| Day 7 | 544 | 10% | Day 7 | 551 | 9% | Day 7 | 547 | 6% | 547 | 8% |
| Day 15 | 540 | 10% | Day 15 | 525 | 9% | Day 15 | 520 | 6% | 528 | 8% |
| Day 22 | 555 | 10% | Day 22 | 572 | 10% | Day 22 | 543 | 6% | 557 | 8% |
| Day 36 | 885 | 16% | Day 36 | 1182 | 20% | Day 36 | 643 | 8% | 903 | 14% |
| Day 149 | 4448 | 81% | Day 163 | 5256 | 88% | Day 149 | 7810 | 91% | 5838 | 87% |

**Table 18 1.5 mg/kg LDP-02 IV**

| Subject # 13 | | | Subject # 15 | | | Subject # 16 | | | Mean | |
|---|---|---|---|---|---|---|---|---|---|---|
| Pre-Dose | 4966 | 100% | Pre-Dose | 5544 | 100% | Pre-Dose | 5622 | 100% | 5378 | 100% |
| 3 hr | 518 | 10% | 3 hr | 539 | 10% | 3 hr | 545 | 10% | 534 | 10% |
| 24 hrs | 482 | 10% | 24 hrs | 487 | 9% | 24 hrs | 520 | 9% | 496 | 9% |
| Day 3 | 511 | 10% | Day 3 | 475 | 9% | Day 3 | 514 | 9% | 500 | 9% |
| Day 7 | 549 | 11% | Day 7 | 535 | 10% | Day 7 | 569 | 10% | 551 | 10% |
| Day 15 | 472 | 9% | Day 15 | 474 | 9% | Day 15 | 491 | 9% | 479 | 9% |
| Day 22 | 603 | 12% | Day 22 | 617 | 11% | Day 22 | 576 | 10% | 599 | 1% |
| Day 36 | 618 | 12% | Day 36 | 866 | 16% | Day 36 | 606 | 11% | 697 | 13% |
| Day 82 | 922 | 19% | Day 80 | 832 | 15% | | | | 877 | 16% |
| Day 134 | 1647 | 33% | Day 134 | 1531 | 28% | | | | 1589 | 30% |
| Day 176 | 2322 | 47% | | | | | | | 1589 | 43% |

**Table 19 2.5 mg/kg LDP-02 IV**

| Subject # 18 | | | Subject # 19 | | | Mean | |
|---|---|---|---|---|---|---|---|
| Pre-Dose | 5922 | 100% | Pre-Dose | 5065 | 100% | 5494 | 100% |
| 3 hr | 527 | 9% | 3 hr | 527 | 10% | 527 | 10% |
| 24 hrs | 568 | 10% | 24 hrs | 571 | 11% | 569 | 10% |
| Day 3 | 511 | 9% | Day 3 | 521 | 10% | 516 | 9% |
| Day 7 | 503 | 9% | Day 7 | 513 | 10% | 508 | 9% |
| Day 15 | 530 | 9% | Day 15 | 544 | 11% | 537 | 10% |
| Day 22 | 588 | 10% | Day 22 | 595 | 12% | 591 | 11% |
| Day 36 | 550 | 9% | Day 36 | 554 | 11% | 552 | 10% |
| Day 70 | 615 | 10% | Day 69 | 566 | 11% | 590 | 11% |
| Day 138 | 4572 | 77% | Day 124 | 1103 | 22% | 2837 | 52% |
| Day 166 | 5603 | 95% | Day 152 | 4094 | 81% | 4849 | 88% |

**Table 20 placebo group**

| | Subject # 1 | | Subject # 7 | | Subject # 11 | | Subject # 14 | | Subject # 17 | |
|---|---|---|---|---|---|---|---|---|---|---|
| Pre-Dose | 5807 | 100% | 5198 | 100% | 8747 | 100% | 7017 | 100% | 5982 | 100% |
| 3 hr | 5630 | 97% | 4305 | 83% | 8454 | 97% | 6208 | 88% | 5520 | 92% |
| 24 hrs | 6672 | 15% | 4347 | 84% | 8033 | 92% | 6699 | 95% | 5410 | 90% |
| Day 3 | 6078 | 105% | 4008 | 77% | 8701 | 99% | 6141 | 88% | 5488 | 92% |
| Day 7 | 5617 | 97% | 4047 | 78% | 8668 | 99% | 6327 | 90% | 5194 | 87% |
| Day 15 | 5797 | 100% | 4758 | 92% | 7516 | 86% | 4851 | 69% | 5759 | 96% |
| Day 22 | 5164 | 89% | 4318 | 83% | 6924 | 79% | 5246 | 75% | 5922 | 99% |
| Day 36 | 6200 | 107% | 4686 | 90% | 7065 | 81% | 7857 | 112% | 5349 | 89% |

### Example 2. Study L297-006

The study entitled, "A Single Dose Phase Ib/IIa, Placebo Controlled, Randomized, Double-Blind Study to Determine the Safety, Tolerability, Pharmacokinetics, Pharmacodynamics, and Effectiveness of LDP-02 in Patients with Moderately Severe Ulcerative Colitis" was completed and certain final results are presented in this section.

### Study Rationale

Results from the Phase I trial (Example 1. Study L297-007) in healthy volunteers showed LDP-02 at doses of 0.15 mg/kg SC and IV, 0.5 mg/kg IV, 1.5 mg/kg IV, and 2.5 mg/kg IV was safe and well-tolerated. In addition, doses of 0.15 mg/kg IV or SC and 0.5 mg/kg IV were shown to have a t_{1/2} of approximately 100 to 130 hours and flow cytometry data showed that unbound α4β7 begins to reappear in the 0.15 mg/kg dosage groups approximately two weeks after dosing. Based upon these data, LDP-02 dosages of 0.15 mg/kg SC, 0.15 mg IV, 0.5 mg/kg N, and 2.0 mg/kg IV were selected for use in the initial study in patients with ulcerative colitis. This study was designed so that each dose of LDP-02 was determined to be safe and well-tolerated prior to escalation to the next dose level.

### Study Design

The study was a randomized, double-blind, placebo-controlled, ascending single-dose study in patients diagnosed with moderately-severe ulcerative colitis. Patients with a documented diagnosis of ulcerative colitis with a minimum disease extent of 25 cm from the anal verge were potentially eligible for the study. Patients with severe ulcerative colitis as defined by Truelove-Witts criteria (Br Med J; 2:1042-1048 (1955)) were excluded. Ulcerative colitis patients who met all inclusion/exclusion criteria were enrolled sequentially into four study groups and, within each study group, were randomly assigned to receive LDP-02 or placebo (i.e., 0.9% sodium chloride). Treatment groups and numbers of patients enrolled are shown in Table 21.

**Table 21: Study Groups**

| Group | Route of Administration* | LDP-02 | | Placebo |
|---|---|---|---|---|
| | | # patients | Dose | # patients |
| 1 | SC | 5 | 0.15 mg/kg | 2 |
| 2 | IV | 5 | 0.15 mg/kg | 2 |
| 3 | IV | 5 | 0.5 mg/kg | 2 |
| 4 | IV | 5 | 2.0 mg/kg | 2 |

Study medication (LDP-02 or placebo) was administered on Day 1 either SC into the thigh or via a 30 minute IV infusion. Safety assessments included recording of adverse events, physical examinations, vital signs, clinical laboratories (i.e., hematology, blood chemistries, and urinalysis), plasma cytokine levels, and ECGs. Blood was drawn at various time points to measure LDP-02 serum concentrations and to assess the effectiveness of LDP-02 to saturate and block α4β7 binding receptors on peripheral blood lymphocytes. The effectiveness of LDP-02 to reduce inflammation in the colon was measured by clinical disease observations, endoscopic appearance, histopathology, and immunohistochemistry.

### Study Results

### Patient Disposition and Demography

Twenty-nine patients with moderately-severe ulcerative colitis were entered into the study and 28 completed the trial. One patient was found to be *Clostridium difficile* toxin positive at screening, but due to a delay in reporting laboratory results the patient was admitted into the trial and received 2.0 mg/kg IV of LDP-02. Once the laboratory results were obtained, the patient was treated with antibiotics and replaced by another patient. There were no other patients discontinued from the study. As patients were recruited into the study over time, there was no attempt to balance the treatment groups with regard to baseline ulcerative colitis history. As such, severity and duration of ulcerative colitis disease and prior medications for ulcerative colitis varied from patient to patient and from treatment group to treatment group. These data are presented in Table 22.

**Table 22: Ulcerative Colitis History by Treatment Group**

| Treatment Group | Time Since Onset of UC Symptoms (yrs)¹ | Time Since Diagnosis of UC (yrs)¹ | # of Acute Exacerbations in past 12 months¹ | Weeks on continuous oral 5-ASA in past 6 months¹ | Weeks on continuous oral steroids in past 6 months¹ |
|---|---|---|---|---|---|
| 0.15 mg/kg SC | 5.32 | 4.6 | 3 | 24.0 | 0 |
| (n=S) | (4.8,6.4) | (4.3,6.4) | (1,12) | (3,26) | (0,6) |
| 0.15 mg/kg IV | 9.58 | 4.9 | 1 | 24.0 | 10 |
| (n=5) | (2.6,14.2) | (2.1,14.0) | (1,3) | (6,26) | (0,24) |
| 0.5 mg/kg IV | 10.8 | 9.0 | 1 | 26.0 | 0 |
| (n=5) | (0.4,11.8) | (0.3,11.8) | (1,2) | (0,26) | (0,15) |
| 2.0 mg/kg IV | 9.34 | 7.65 | 2 | 25.0 | 5 |
| (n=6) | (3.4,58.8) | (3.2,19.4) | (1,5) | (0,26) | (0,26) |
| All LDP-02 | 5.99 | 4.9 | 2 | 26.0 | 0 |
| (n=21) | (0.4,58.8) | (0.3,19.4) | (1,12) | (0,26) | (0,26) |
| Placebo | 5.27 | 4.85 | 1.5 | 24.0 | 16 |
| (n=8) | (0.4,11.0) | (0.3,9.7) | (1,4) | (0,26) | (0,26) |

| | | | | | |
|---|---|---|---|---|---|
| ¹Median values | | | | | |

### Disease Measurements

Although.this was primarily a dose-ranging safety and pharmacokinetics study, various parameters were measured to assess effectiveness of treatment. Effectiveness assessments included recording changes from baseline using a modified Baron's (endoscopy) Scoring System, the Mayo Clinic Disease Activity Index Score, the Powell-Tuck Disease Activity Index Score, stool frequency, and the Inflammatory Bowel Disease Questionnaire. Changes from baseline to Day 30 for these parameters are shown in Table 23. For patients in which there was no Day 30 evaluation, the last post-baseline observation obtained was carried forward to Day 30.

**Table 23: Change from Baseline to Day 30 in Disease Parameters**

| Treatment Group | Change from baseline to Dray 30¹ | | | | |
|---|---|---|---|---|---|
| | Endoscopic Severity Score | Mayo Clinic Activity Index | Powell-Tuck Activity Index | Stool Frequency | Total IBDQ |
| 0.15 mg/kg SC | 0 | -3.0 | -3.0 | -1.0 | 14.0 |
| (n=5) | (-2,0) | (-9,0) | (-6,-2) | (-7,1) | (14,72) |
| 0.15 mg/kg IV | 0 | -1.0 | 0 | -0.4 | 8.0 |
| (n=5) | (0,1) | (-3,2) | (-3,3) | (-5,2) | (-3,95) |
| 0.5 mg/kg IV | -2.0 | -10 | -6.0 | -5.3 | 37.0 |
| (n=5) | (-3,0) | (-11,0) | (-13,-2) | (-6,0) | (14,80) |
| 2.0 mg/kg IV | -0.5 | -2.0 | -1.5 | -3.2 | -2.5 |
| (n=6) | (-2,1) | (-6,3) | (-5,-5) | (-8,2) | (-59,95) |
| All LDP-02 | 0 | -3.0 | -3.0 | -2.4 | 14.0 |
| (n=21) | (-3,1) | (-11,3) | (-13,5) | (-8,2) | (-59,95) |
| Placebo | -1.0 | -5.0 | -6.0 | -3.2 | 53.5 |
| (n=8) | (-3,2) | (-8.4) | (-9,-4) | (-12,2) | (-30,82) |

| | | | | | |
|---|---|---|---|---|---|
| ¹Median values and range. For patients without a Day 30 evaluation the last post-baseline evaluation was carried forward to Day 30. | | | | | |

As seen from the results presented in Table 23, there was variability in response among the different treatment groups. The patients receiving 0.5 mg/kg IV appeared to have the best responses; the median endoscopic severity score was reduced by two grades and the Mayo Clinic score was reduced by 10 points with a decrease in stool frequency. Three of the five patients receiving 0.5 mg/kg IV had a two point improvement in the modified Baron sigmoidoscopy score which is considered an endoscopic response; only one patient (compared with a total of five treated per group) in both the 2.0 mg/kg IV and 0.15 mg/kg SC groups had an endoscopic response. The placebo group also experienced an improvement in sigmoidoscopic score and Mayo Clinic score, although both were less in magnitude when compared to the 0.5 mg/kg IV group. Two of the eight patients experienced an endoscopic response.

The number of patients with a complete remission, defined as a zero on the modified Baron sigmoidoscopic score and on the Mayo Clinic score at Day 30, are reported in Table 24.

**Table 24: Patients in Complete Remission at Day 30**

| Treatment Group | Measured at Day 30¹ | |
|---|---|---|
| | Number of Patients with Complete Remission | Percentage of Patients in Complete Remission |
| 0.15 mg/kg SC | 0 | 0 |
| (n=5) | | |
| 0.15 mg/kg IV | 0 | 0 |
| (n=5) | | |
| 0.5 mg/kg IV | 2 | 40% |
| (n=5) | | |
| 2.0 mg/kg IV | 0 | 0 |
| (n=6) | | |
| All LDP-02 | 2 | 9.5% |
| (n=21) | | |
| Placebo | 0 | 0 |
| (n=8) | | |

| | | |
|---|---|---|
| ¹Zero on the modified Baron Score and the Mayo Clinic Score in Day 30 results | | |

None of the patients in the placebo group experienced a complete remission while two patients among those receiving LDP-02 had complete remissions. The two patients both were in the same group; both patients received a single administration of 0.5 mg/kg of LDP-02. One of the patients was receiving concurrent mesalamine therapy, while the other was receiving concurrent low dose corticosteroid (20 mg prednisone per day orally).

### Pharmacokinetics

The assay of LDP-02 in serum was performed by Cytometry Associates, Inc. as previously described (Study L297-007). Blood samples were collected prior to and immediately following the completion of infusion (Day 1) and on Days 2, 3, 5, 10, 14, 21, 30 and 60 to assess the pharmacokinetic profile of LDP-02.

LDP-02 concentrations over time by individual patient and mean pharmacokinetic parameters by LDP-02 dose are presented in the Appendix to study L296-006.

As seen in FIG. 8, serum levels of LDP-02 for the 0.15 mg/kg IV and SC groups fall to <1.0 (µg/mL approximately 20 days post-dose. For the 2.0 mg/kg dose group, LDP-02 levels remain elevated out to approximately Day 60. Table 25 presents the key pharmacokinetic parameters by treatment group.

**Table 25: Pharmacokinetic Parameters of LDP-02**

| | Dose and Route of Administration of LDP-02 (number of subjects with data)² | | | |
|---|---|---|---|---|
| Pharmacokinetic Parameter¹ | 0.15 mg/kg SC (n=5) | 0. 15 mg/kg IV | 0. 5 mg/kg IV | 2. 0 mg/kg IV |
| | | (n=5) | (n=5) | (n=4)³ |
| Cₘₐₓ (µg/mL) | 1.44 | 3.602 | 10.544 | 32.933 |
| | (0.33) | (0.958) | (2.582) | (3.360) |
| tₘₐₓ (days) | 5 | 0.13 | 0.13 | 0.13 |
| (median & range) | (3-10) | (0.13-0.13) | (0.13-0.13) | (0.13-2) |
| t_{1/2z} (days) | 15.63 | 18.91 | 10.62 | 15.0 |
| | (15.92) | (20.97) | (5.23) | (5.36) |
| AUCₐₗₗ | 25 | 27 | 91 | 515 |
| (µg·day/mL) | (16) | (11) | (32) | (93) |
| λ_{z} (1/day) | 0.1226 | 0.0879 | 0.0927 | 0.0542 |
| | (0.1064) | (0.0757) | (0.0775) | (0.0298) |
| AUC(INF) | 31 | 34 | 100 | 553 |
| (µg·day/mL) | (23) | (18) | (39) | (116) |
| CL⁴ | 9.21 | 7.75 | 6.06 | 2.31 |
| (mLday/kg) | (9.54) | (1.93) | (1.32) | (1.19) |
| V_{z}⁴ (mL/kg) | 95.08 | 101.05 | 77.63 | 76.64 |
| | (54.19) | (62.87) | (30.90) | (20.03) |

| | | | | |
|---|---|---|---|---|
| ¹All values are mean +/- SD unless otherwise indicated. The SD appears in parenthesis. ²Two patients, one in the 0.15 mg/kg SC and one in the 0.5 mg/kg IV groups had evaluable data through Study Day 21 with measurement at later times which were not physiologically possible. ³One patient in the 2.0 mg/kg IV dosing group was withdrawn at Study Day 10 and had a surgical intervention. The pharmacokinetic results for this patient are not included. ⁴Clearance and volume terms for the SC dose group are the apparent clearance (CL/F) and apparent volume (V_{z}/F). | | | | |

There does appear to be linearity with dose for the maximum concentration of LDP-02 and the area under the curve measured after IV administration. The clearance and the terminal elimination half life appear to be independent of IV dose administered. The volume of distribution appears to decrease slightly with increasing doses of IV LDP-02.

### Assessment of the Pharmacodynamic Effect of LDP-02

FACS analysis to measure the presence of α4β7 sites on blood lymphocytes was previously described (Study L296-007). Serum α4β7 binding over time (i.e., MESF values and percentage of baseline at each post-dose time point) are presented by individual patient and by treatment group in the Appendix to Study L297-006.

Mean percent of baseline MESF over time for all treatments are presented in FIG. 9. As seen in FIG. 9, percent of baseline MESF rapidly falls to approximately 10% after SC and IV administration of LDP-02 with duration of effect dependent upon dose. Starting at about day 10, α4β7 signal started to return to baseline for the 0.15 mg/kg IV and SC dose groups. However, α4β7 signal started to return to baseline between day 30 and day 60 for the 0.5 mg/kg IV and 2.0 mg/kg dose groups.

### Conclusions

Administration of LDP-02 at doses of 0.15 mg/kg IV and SC, 0.5 mg/kg IV, and 2.0 mg/kg IV to patients with moderately-severe ulcerative colitis was well-tolerated.

The pharmacokinetic and pharmacodynamic data from patients with ulcerative colitis were consistent with those found in healthy volunteers. There appeared to be linearity with dose for the maximum concentration of LDP-02 and area under the curve measured after IV administration. The clearance and the terminal elimination half life appeared to be independent of IV dose administration. The volume of distribution appeared to decrease slightly with increasing doses of IV LDP-02. The percent of baseline MESF declined to ~10% rapidly after SC and IV administration of LDP-02 with duration of effect dependent upon dose. For the 0.15 mg/kg IV and SC dose groups, percent of baseline MESF started returning to baseline approximately 10 days after dosing whereas this started to occur at -30 days and -60 days for the 0.5 mg/kg IV and 2.0 mg/kg dose groups, respectively.

### Appendix to Study L297-006

LDP-02 Serum Concentration Over Time by Subject by Treatment Group. Data obtained from individual subjects are presented in Tables 26-30. The data presented in Tables 26-30 are in µg/mL.

**Table 26 Group 1: 0.15 mg/kg LDP-02 SC**

| Time (day) | Subject # 201101 | Subject # 301103 | Subject # 302105 | Subject # 304107 | Subject # 401104 |
|---|---|---|---|---|---|
| Pre-Dose | BQL | BQL | BQL | BQL | BQL |
| 0.125 | BQL | 0.07 | BQL | BQL | NS |
| 2 | 0.61 | 0.91 | 0.94 | 1.01 | 1.29 |
| 3 | 0.90 | 1.10 | 1.29 | 1.49 | 1.65 |
| 5 | 0.76 | 1.48 | NR | 1.66 | 1.74 |
| 10 | 0.15 | 1.12 | 1.40 | 0.92 | 1.44 |
| 14 | BQL | 0.61 | 0.78 | 0.24 | 0.99 |
| 21 | BQL | BQL | NS | 0.11 | 0.65 |
| 30 | BQL | 0.33 | 0.84 | 0.26 | 0.12 |
| 60 | BQL | 0.23 | 0.37 | 0.30 | BQL |

| | | | | | |
|---|---|---|---|---|---|
| BQL= reported as non-detectable NS= no sample received from laboratory | | | | | |

**Table 27 Group 2: 0.15 mg/kg LDP-02 IV**

| Time (Day) | Subject # 101201 | Subject # 102202 | Subject # 305204 | Subject # 402203 | Subject # 403206 |
|---|---|---|---|---|---|
| Pre-Dose | BQL | BQL | BQL | BQL | BQL |
| 0.125 | 4.14 | 4.88 | 3.35 | 2.34 | 3.30 |
| 2 | NR | 2.74 | 1.92 | 1.83 | 2.34 |
| 3 | 3.12 | 3.15 | 1.55 | 1.42 | 2.03 |
| 5 | 1.82 | 1.83 | 1.33 | 0.82 | 1.19 |
| 10 | 0.81 | 0.88 | 0.86 | 0.37 | 0.79 |
| 14 | 0.32 | 0.15 | BQL | 0.23 | 0.26 |
| 21 | 0.38 | 0.12 | 0.10 | BQL | BQL |
| 30 | 0.38 | BQL | 0.40 | BQL | 0.05 |
| 60 | 0.24 | BQL | 0.36 | BQL | 0.14 |

| | | | | | |
|---|---|---|---|---|---|
| NR= no sample result reported from laboratory | | | | | |

**Table 28 Group 3: 0.5 mg/kg LDP-02 IV**

| Time (day) | Subject # 206302 | Subject # 208303 | Subject # 309306 | Subject # 502304 | Subject # 503307 |
|---|---|---|---|---|---|
| Pre-Dose | BQL | BQL | BQL | BQL | BQL |
| 0.125 | 14.06 | 12.33 | 7.90 | 8.67 | 9.76 |
| 2 | 10.01 | 8.51 | 5.73 | 5.84 | 8.26 |
| 3 | 6.56 | 6.45 | 4.96 | 4.67 | 7.27 |
| 5 | 4.15 | 5.52 | 3.59 | 2.94 | 5.61 |
| 10 | 3.17 | 4.46 | 2.81 | 3.11 | 4.21 |
| 14 | 2.51 | 0.14 | 2.46 | 1.14 | 3.01 |
| 21 | BQL | 0.17 | *0.14 | BQL | 2.04 |
| 30 | BQL | 0.48 | BQL | 0.06 | 1.29 |
| 60 | 0.41 | 1.73 | 0.10 | 0.28 | BQL |

**Table 29 Group 4: 2.0 mg/kg LDP-02 IV**

| Time (day) | Subject # 104403 | Subject # 210402 | Subject # 310415 | Subject # 404401 | Subject # 504405 | Subject # 506407 |
|---|---|---|---|---|---|---|
| Pre-Dose | BQL | BQL | BQL | BQL | BQL | BQL |
| 0.125 | 30.45 | 38.83 | 37.66 | 29.71 | 28.90 | 32.18 |
| 2 | 32.18 | 28.22 | 35.14 | 27.49 | 27.49 | 26.87 |
| 3 | 23.93 | 17.40 | 27.49 | 24.45 | 22.92 | 22.46 |
| 5 | 21.52 | 15.34 | 21.52 | 18.42 | 21.52 | 17.79 |
| 10 | 13:10 | 41.11 | 14.82 | 13.10 | 10.99 | 11.96 |
| 14 | 11.72 | 3.13 | 13.10 | 11.23 | 1.22 | 9.03 |
| 21 | 7.53 | 0.08 | 10.99 | 8.55 | 0.12 | 5.70 |
| 30 | 5.80 | BQL | 8.26 | 7.02 | NR | 4.19 |
| 60 | 1.71 | 0.41 | 2.24 | 1.95 | NR | 0.06 |

**Table 30 placebo group**

| Time (day) | Subject # 202102 | Subject # 303106 | Subject # 103205 | Subject # 306207 | Subject # 308305 | Subject # 501301 | Subject # 209404 | Subject # 505406 |
|---|---|---|---|---|---|---|---|---|
| Pre-Dose | BQL | BQL | BQL | BQL | BQL | BQL | BQL | BQL |
| 0.125 | BQL | BQL | BQL | BQL | BQL | BQL | BQL | BQL |
| 2 | BQL | BQL | BQL | BQL | BQL | BQL | BQL | BQL |
| 3 | BQL | BQL | BQL | BQL | BQL | BQL | BQL | BQL |
| 5 | BQL | BQL | BQL | BQL | BQL | BQL | BQL | BQL |
| 10 | BQL | BQL | BQL | BQL | BQL | BQL | BQL | BQL |
| 14 | BQL | BQL | BQL | BQL | BQL | BQL | BQL | BQL |
| 21 | BQL | BQL | NR | BQL | BQL | BQL | BQL | BQL |
| 30 | BQL | BQL | BQL | BQL | BQL | BQL | BQL | BQL |
| 60 | BQL | BQL | BQL | BQL | BQL | BQL | BQL | BQL |

| | | | | | | | | |
|---|---|---|---|---|---|---|---|---|
| BQL = below quantitation limit. | | | | | | | | |

Mean Pharmacokinetic Parameters by Treatment Group. Data obtained from individual subjects are presented in Tables 31-34.

**Table 31 Group 1: 0.15 mg/kg LDP-02 SC**

| Subject | Cₘₐₓ (µg/mL) | tₘₐₓ (days) | t_{½z} (days) | AUCₐₗₗ (µg·day/m L) | λ_{z} (1/day) | AUC (µg·day/mL) | CL (mL/day/kg) | V_{z} (mL/kg) |
|---|---|---|---|---|---|---|---|---|
| 201101 | 0.90 | 3 | 2.58 | 5.30 | 0.2692 | 5.86 | 25.61 | 95.15 |
| 301103 | 1.48 | 5 | 34.61 | 30.39 | 0.0200 | 41.87 | 3.58 | 178.87 |
| 302105 | 1.40 | 10 | 31.35 | 46.94 | 0.0221 | 63.68 | 2.36 | 106.55 |
| 304107 | 1.66 | 5 | 3.88 | 15.41 | 0.1788 | 16.02 | 9.36 | 52.37 |
| 401104 | 1.74 | 5 | 5.72 | 28.17 | 0.1212 | 29.16 | 5.14 | 42.45 |
| Mean | 1.436 | 5.6 | 15.628 | 25.242 | 0.1223 | 31.318 | 9.21 | 95.078 |
| SD | 0.329 | 2.607 | 15.921 | 15.813 | 0.1064 | 22.613 | 9.54 | 54.190 |

| | | | | | | | | |
|---|---|---|---|---|---|---|---|---|
| Cₘₐₓ = maximum concentration tₘₐₓ = time to maximum concentration λ_{z} = a measure of elimination t_{½z} = terminal half-live AUCₜ = AUC ₐₗₗ = area under the curve using all time points AUC = AUCₑₓₜ = area under curve extrapolated AUC ext (%) = % of area under curve attributed to extrapolation extrapolation V_{z} = apparent volume of distribution CL = Clearance | | | | | | | | |

**Table 32 Group 2: 0.15 mg/kg LDP-02 IV**

| Subject | Cₘₐₓ (µg/mL) | tₘₐₓ (days) | t_{½z} (days) | AUCₐₗₗ (µg·day/mL) | λ_{z} (1/day) | AUC (µg·day/mL) | CL (mL/day/kg) | V_{z} (mL/kg) |
|---|---|---|---|---|---|---|---|---|
| 101201 | 4.14 | 0.13 | 54.69 | 39.64 | 0.0127 | 58.58 | 2.56 | 202.06 |
| 102202 | 4.88 | 0.13 | 3.62 | 25.15 | 0.1914 | 25.78 | 5.82 | 30.39 |
| 305204 | 3.35 | 0.13 | 19.37 | 34.17 | 0.0358 | 44.23 | 3.39 | 94.77 |
| 402203 | 2.34 | 0.13 | 4.88 | 12.10 | 0.1420 | 13.72 | 10.94 | 77.03 |
| 403206 | 3.30 | 0.13 | 11.99 | 23.28 | 0.0578 | 25.70 | 5.84 | 100.99 |
| Mean | 3.602 | 0.13 | 18.91 | 26.868 | 0.0879 | 33.602 | 5.71 | 101.05 |
| SD | 0.9579 | 0 | 20.97 | 10.611 | 0.0757 | 17.718 | 3.27 | 62.87 |

**Table 33 Group 3: 0.5 mg/kg LDP-02 IV**

| Subject | Cₘₐₓ (µg/mL) | tₘₐₓ (days) | t_{½z} (days) | AUCₐₗₗ (µg·day/mL) | λ_{z} (1/day) | AUC (µg·day/mL) | CL (mL/day/kg) | V_{z} (mL/kg) |
|---|---|---|---|---|---|---|---|---|
| 206302 | 14.06 | 0.13 | 17.21 | 139.26 | 0.0403 | 149.44 | 3.35 | 83.08 |
| 208303 | 12.33 | 0.13 | 3.02 | 74.99 | 0.2293 | 75.73 | 6.60 | 28.79 |
| 309306 | 7.90 | 0.13 | 9.22 | 67.49 | 0.0751 | 68.82 | 7.27 | 96.69 |
| 502304 | 8.67 | 0.13 | 10.52 | 65.34 | 0.0659 | 69.59 | 7.19 | 109.09 |
| 503307 | 9.76 | 0.13 | 13.11 | 109.80 | 0.0529 | 134.20 | 3.73 | 70.48 |
| Mean | 10.544 | 0.13 | 10.616 | 91.376 | 0.0927 | 99.556 | 5.628 | 77.626 |
| SD | 2.582 | 0 | 5.229 | 32.207 | 0.0775 | 39.048 | 1.928 | 30.90 |

**Table 34 Group 4: 2.0 mg/kg LDP-021V**

| Subject | Cₘₐₓ (µg/mL) | tₘₐₓ (days) | t_{½z} (days) | AUCₐₗₗ (µg·day/mL) | λ_{z} (1 day) | AUC (µg·day/mL) | CL (mL/day/kg) | V_{z} (mL/kg) |
|---|---|---|---|---|---|---|---|---|
| 104403 | 32.18 | 2.00 | 17.92 | 510.32 | 0.0387 | 554.52 | 3.61 | 93.22 |
| 310415 | 37.66 | 0.13 | 16.72 | 626.06 | 0.0415 | 680.08 | 2.94 | 70.92 |
| 404401 | 29.71 | 0.13 | 18.34 | 525.63 | 0.0378 | 577.22 | 3.46 | 91.68 |
| 506407 | 32.18 | 0.13 | 7.02 | 398.45 | 0.0988 | 399.06 | 5.01 | 50.75 |
| 506407 Mean | 32.933 | 0.13 | 15.0 | 515.12 | 0.0542 | 552.72 | 3.755 | 76.643 |
| SD | 3.360 | 0.935 | 5.364 | 93.19 | 0.0298 | 116.10 | 0.885 | 20.034 |

Serum α4β7 Binding Over Time by Subject by Treatment Group. Data obtained from individual subjects are presented in Tables 35-40. For each subject the time of blood sampling, MESF of the sample and % of baseline (pre-dose) MESF is presented.

**Table 35 Group 1: 0.15 mg/kg LDP-02 SC**

| Time Days | Subject # 201101 | | Subject # 301103 | | Subject # 302105 | | Subject # 304107 | | Subject # 401104 | | Mean | |
|---|---|---|---|---|---|---|---|---|---|---|---|---|
| Pre-Dose | 10046 | 100% | 7326 | 100% | 12684 | 100% | 13117 | 100% | 3369 | 100% | 9308 | 100% |
| 0.125 | 951 | 9% | 762 | 10% | 1700 | 13% | 857 | 7% | 1105 | 33% | 1075 | 12% |
| 3 | 797 | 8% | 383 | 5% | 707 | 6% | 853 | 7% | 575 | 17% | 663 | 17% |
| 5 | 845 | 8% | 723 | 10% | | | 815 | 16% | 1052 | 31% | 859 | 9% |
| 10 | 675 | 7% | 717 | 10% | 862 | 7% | 865 | 7% | 941 | 28% | 812 | 9% |
| 14 | 4197 | 42% | 754 | 10% | 830 | 7% | 905 | 7% | 1058 | 31% | 1549 | 17% |
| 21 | 9610 | 96% | 803 | 11% | 834 | 7% | 3443 | 26% | 948 | 28% | 3128 | 34% |
| 30 | 9462 | 94% | 1142 | 16% | 1275 | 10% | 1587 | 12% | 1113 | 33% | 2916 | 31% |
| 60 | 9839 | 98% | 752 | 10% | 849 | 7% | 1262 | 10% | 2849 | 85% | 3110 | 33% |

**Table 36 Group 2: 0.15 mg/kg LDP-02 IV**

| Time Days | Subject # 101201 | | Subject # 102202 | | Subject # 305204 | | Subject # 402203 | | Subject # 403206 | | Mean | |
|---|---|---|---|---|---|---|---|---|---|---|---|---|
| Pre-Dose | 2588 | 100% | 2712 | 100% | 8394 | 100% | 10016 | 100% | 8342 | 100% | 6410 | 100% |
| 0.125 | 701 | 27% | 827 | 30% | 848 | 10% | 642 | 6% | 875 | 10% | 779 | 12% |
| 3 | 760 | 29% | 784 | 29% | 820 | 10% | 679 | 7% | 875 | 10% | 784 | 12% |
| 5 | 677 | 26% | 884 | 33% | 1012 | 12% | 639 | 6% | 859 | 10% | 814 | 13% |
| 10 | 671 | 26% | 753 | 28% | 943 | 11% | 690 | 7% | 856 | 10% | 783 | 12% |
| 14 | 1008 | 39% | 1515 | 56% | 1377 | 16% | 608 | 6% | 744 | 9% | 1050 | 16% |
| 21 | 953 | 37% | 4220 | 156% | 1860 | 22% | 2044 | 20% | 1606 | 19% | 2137 | 33% |
| 30 | 988 | 38% | 328 | 12% | 2332 | 28% | 3302 | 33% | 2560 | 31% | 1902 | 30% |
| 60 | 1680 | 65% | 3670 | 135% | 3275 | 39% | 6851 | 68% | 1168 | 14% | 3329 | 52% |

**Table 37 Group 3: 0.5 mg/kg LDP-02 IV**

| Time Days | Subject # 206302 | | Subject # 208303 | | Subject # 309306 | | Subject # 502304 | | Subject # 503307 | | Mean | |
|---|---|---|---|---|---|---|---|---|---|---|---|---|
| Pre-Dose | 3830 | 100% | 11267 | 100% | 5084 | 100% | 5615 | 100% | 9400 | 100% | 7039 | 100% |
| 10.125 | 1322 | 35% | 1577 | 14% | 887 | 17% | 879 | 16% | 1021 | 11% | 1137 | 16% |
| 3 | 1189 | 31% | 2012 | 18% | 914 | 18% | 775 | 14% | 982 | 10% | 1174 | 17% |
| 5 | 1054 | 28% | 1717 | 15% | 962 | 19% | 809 | 14% | 1147 | 12% | 1138 | 16% |
| 10 | 1195 | 31% | 2108 | 19% | 965 | 19% | 829 | 15% | 732 | 8% | 1166 | 17% |
| 14 | 1339 | 35% | 2405 | 21% | 1106 | 22% | 610 | 11% | 801 | 9% | 1252 | 18% |
| 21 | 1296 | 34% | 2085 | 19% | 671 | 13% | 636 | 11% | 733 | 8% | 1084 | 15% |
| 30 | 1483 | 39% | 1706 | 15% | 1203 | 24% | 860 | 15% | 611 | 7% | 1173 | 17% |
| 60 | 985 | 26% | 1038 | 9% | 1611 | 32% | 764 | 14% | 7611 | 81% | 2402 | 34% |

**Table 38 Group 4: 2.0 mg/kg LDP-02 IV***

| Time Days | Subject # 104403 | | Subject # 210402 | | Subject # 310415 | | Subject # 404401 | | Subject # 506407 | | Mean | |
|---|---|---|---|---|---|---|---|---|---|---|---|---|
| Pre-Dose | 6714 | 100% | 5026 | 100% | 4642 | 100% | 4235 | 100% | 7418 | 100% | 5607 | 100% |
| 0.125 | 695 | 10% | 666 | 13% | 736 | 16% | 671 | 16% | 738 | 10% | 701 | 13% |
| 3 | 659 | 10% | 671 | 13% | 632 | 14% | 760 | 18% | 683 | 9% | 681 | 12% |
| 5 | 633 | 9% | 659 | 13% | 663 | 14% | 730 | 17% | 665 | 9% | 670 | 12% |
| 10 | 703 | 10% | 636 | 13% | 556 | 12% | 778 | 18% | 734 | 10% | 681 | 12% |
| 14 | 681 | 10% | 590 | 12% | 640 | 14% | 658 | 16% | 755 | 10% | 665 | 12% |
| 21 | 528 | 8% | 621 | 12% | 568 | 12% | 586 | 14% | 756 | 10% | 612 | 11% |
| 30 | 639 | 10% | 1218 | 24% | 599 | 13% | 682 | 16% | 740 | 10% | 776 | 14% |
| 60 | | | | | | | | | | | | |

| | | | | | | | | | | | | |
|---|---|---|---|---|---|---|---|---|---|---|---|---|
| *No data for Subject # 505405 | | | | | | | | | | | | |

**Table 39 Placebo Group**

| Time Days | Subject # 202102 | | Subject # 303106 | | Subject # 103205 | | Subject # 306207 | | Subject # 308305 | | Subject # 501301 | |
|---|---|---|---|---|---|---|---|---|---|---|---|---|
| Pre-Dose | 7657 | 100% | 21074 | 100% | 4935 | 100% | 8070 | 100% | 15162 | 100% | 5274 | 100% |
| 0.125 | 5643 | 74% | 23312 | 111% | 4935 | 100% | 6837 | 85% | 15162 | 100% | 6424 | 122% |
| 3 | 8831 | 115% | 19528 | 93% | 4593 | 93% | 7162 | 89% | 13876 | 92% | 6022 | 114% |
| 5 | 7158 | 93% | 16567 | 79% | 4452 | 90% | 5044 | 63% | 13094 | 86% | 5530 | 105% |
| 10 | 7413 | 97% | 17575 | 83% | 5499 | 111% | 4750 | 59% | 14531 | 96% | 8201 | 155% |
| 14 | 6092 | 80% | 17827 | 85% | 3222 | 65% | 4169 | 52% | 10294 | 68% | 6740 | 128% |
| 21 | 8463 | 111% | 18048 | 86% | | | 4491 | 56% | 12700 | 84% | 7205 | 137% |
| 30 | 7353 | 96% | 15817 | 75% | 2317 | 47% | 11458 | 142% | 9328 | 62% | 5745 | 109% |
| 60 | 3385 | 44% | 11810 | 56% | | | 4771 | 59% | 9648 | 64% | 3262 | 62% |

**Table 40 Placebo group**

| Time Days | Subject # 209404 | | Subject # 505406 | | Mean | |
|---|---|---|---|---|---|---|
| Pre-Dose | 11012 | 100% | 7579 | 100% | 10095 | 100% |
| 0.125 | 11826 | 107% | 9025 | 119% | 10396 | 103% |
| 3 | 10549 | 96% | 8792 | 116% | 9919 | 98% |
| 5 | 11614 | 105% | 6217 | 82% | 8710 | 86% |
| 10 | 8238 | 75% | 7150 | 94% | 9170 | 91% |
| 14 | 8382 | 76% | 4787 | 63% | 7689 | 76% |
| 21 | 7031 | 64% | 7160 | 94% | 9300 | 92% |
| 30 | 6817 | 62% | 8166 | 108% | 8375 | 83% |
| 60 | | | | | | |

## Claims

1. Use of a humanized immunoglobulin or antigen-binding fragment thereof, for the manufacture of a medicament for treating a human having inflammatory bowel disease, wherein said medicament is for administering in a single dose or in an initial dose followed by one or more subsequent doses wherein no more than about 8 mg immunoglobulin or fragment per kg body weight are administered during a period of about one month, and
wherein said humanized immunoglobulin or antigen-binding fragment thereof comprises an antigen binding region of nonhuman origin and at least a portion of an antibody of human origin and has binding specificity for the α4β7 complex, and wherein said antigen-binding region comprises three complementarity determining regions (CDR1, CDR2 and CDR3) of a light chain variable region and three-complementarity determining regions (CDR1, CDR2 and CDR3) of a heavy chain variable region of the amino acid sequence set forth below:
| | |
|---|---|
| light chain: | CDR1 SEQ ID NO:9 |
| | CDR2 SEQ ID NO:10 |
| | CDR3 SEQ ID NO:11 |
| heavy chain: | CDR1 SEQ ID NO:12 |
| | CDR2 SEQ ID NO:13 |
| | CDR3 SEQ ID NO:14. |

2. The use of Claim 1, wherein said humanized immunoglobulin or antigen-binding fragment thereof comprises the heavy chain variable region of SEQ ID NO:6.

3. The use of Claim 1 or 2, wherein said humanized immunoglobulin or antigen-binding fragment thereof comprises the light chain variable region of SEQ ID NO:8.

4. The use of Claim 1, wherein said medicament is sufficient to achieve and maintain a) about 50% or greater saturation of α4β7 integrin binding sites on circulating lymphocytes and/or b) about 50% or greater inhibition of α4β7 integrin expression on the cell surface of circulating lymphocytes, and wherein said saturation and/or inhibition is maintained for a period of at least about 10 days following administration of said dose.

5. The use of Claim 4, wherein each of said doses independently comprise an amount of humanized immunoglobulin or antigen-binding fragment which is sufficient to achieve a) about 60% or greater saturation of α4β7 integrin binding sites on circulating lymphocytes and/or b) about 60% or greater inhibition of α4β7 integrin expression on the cell surface of circulating lymphocytes.

6. The use of Claim 4, wherein each of said doses independently comprise an amount of humanized immunoglobulin or antigen-binding fragment which is sufficient to achieve a) about 70% or greater saturation of α4β7 integrin binding sites on circulating lymphocytes and/or b) about 70% or greater inhibition of α4β7 integrin expression on the cell surface of circulating lymphocytes.

7. The use of Claim 4, wherein each of said doses independently comprise an amount of humanized immunoglobulin or antigen-binding fragment which is sufficient to achieve a) about 80% or greater saturation of α4β7 integrin binding sites on circulating lymphocytes and/or b) about 80% or greater inhibition of α4β7 integrin expression on the cell surface of circulating lymphocytes.

8. The use of Claim 4, wherein each of said doses independently comprise an amount of humanized immunoglobulin or antigen-binding fragment which is sufficient to achieve and maintain said saturation and/or inhibition for a period of at least about 14 days, or at least about 20 days, or at least about 25 days, or at least about 30 days, or at least about 60 days following administration of said dose.

9. The use of Claim 1, wherein each of said doses comprises an amount of humanized immunoglobulin which is sufficient to achieve and maintain a serum concentration of humanized immunoglobulin of at least about I µg/mL for a period of at least about 10 days, or at least about 14 days, or at least about 20 days, or at least about 25 days, or at least about 30 days, or at least about 60 days following administration of said dose.

10. The use of any preceding Claim, wherein each of said doses independently comprise about 0.1 to about 8 mg humanized immunoglobulin or antigen-binding fragment per kg body weight.

11. The use of any preceding Claim, wherein each of said doses independently comprise about 0.1 to about 5 mg humanized immunoglobulin or antigen-binding fragment per kg body weight.

12. The use of any preceding Claim, wherein each of said doses independently comprise about 0.1 to about 2.5 mg humanized immunoglobulin or antigen-binding fragment per kg body weight.

13. The use of any preceding Claim, wherein each of said doses independently comprise about 0.15, about 0.5, about 1.0, about 1.5 or about 2.0 mg humanized immunoglobulin or antigen-binding fragment per kg body weight.

14. The use of any preceding Claim, wherein the interval between the initial dose and subsequent doses is at least about 14 days, or at least about 21 days, or at least about 28 days, or at least about 30 days.

15. The use of any preceding Claim, further comprising use of an effective amount of one or more additional therapeutic agents in the manufacture of the medicament.

16. The use of Claim 15, wherein said agents are selected from the group consisting of steroids, immunosuppressive agents, non-steroidal anti-inflammatory agents and immunomodulators.

17. The use of Claim 15, wherein said agents are selected from the group consisting of azathioprene, 6-mercaptopurine, sulfasalazine, 5-amino salicylic acid, prednisone and prednisolone.

18. The use of any preceding Claim, wherein said inflammatory bowel disease is ulcerative colitis.

19. The use of Claim 18, wherein said inflammatory bowel disease is Crohn's disease

20. The use of claim 1, wherein the minimum interval between any two doses is a period of at least about 1 week.

21. Use of a humanized immunoglobulin or antigen-binding fragment thereof, for the manufacture of a medicament for inhibiting relapse and/or recurrence of quiescent inflammatory bowel disease wherein said medicament is for administering in a single dose or in an initial dose and subsequent doses wherein no more than about 8 mg immunoglobulin or fragment per kg body weight are administered during a period of about 30 days and wherein said humanized immunoglobulin or antigen-binding fragment thereof comprises an antigen-binding region of nonhuman origin and at least a portion of an antibody of human origin and has binding specificity for the α4β7 complex, and wherein said antigen-binding region comprises three complementarity determining regions (CDR1, CDR2 and CDR3) of a light chain variable region and three-complementarity determining regions (CDR1, CDR2 and CDR3) of a heavy chain variable region of the amino acid sequence set forth below:
| | |
|---|---|
| light chain: | CDR1 SEQ ID NO:9 |
| | CDR2 SEQ ID NO:10 |
| | CDR3 SEQ ID NO:11 |
| heavy chain: | CDR1 SEQ ID NO:12 |
| | CDR2 SEQ ID NO:13 |
| | CDR3 SEQ ID NO:14. |

22. The use of Claim 21, wherein quiescence has been induced by medical or surgical therapy or wherein said inflammatory bowel disease is ulcerative colitis or Crohn's disease.

23. The use of Claim 1 or 4, wherein each of said doses independently comprise about 6 mg immunoglobulin or fragment per kg body weight.

24. The use of Claim 1 or 4, wherein the interval between doses is at least about 50 days.

## Patentansprüche

1. Verwendung eines humanisierten Immunglobulins oder Antigen bindenden Fragments davon zur Herstellung eines Medikaments zur Behandlung eines an entzündlicher Darmerkrankung leidenden Menschen, wobei das Medikament zur Verabreichung in einer einzigen Dosis oder einer anfänglichen Dosis gefolgt von einer oder mehreren Dosen gedacht ist, wobei während einer Zeitspanne von etwa 1 Monat nicht mehr als etwa 8 mg Immunglobulin oder Fragment pro Kg Körpergewicht verabreicht werden, und
wobei das humanisierte Immunglobulin oder Antigen bindende Fragment davon einen Antigen bindenden Bereich nicht-humanen Ursprungs und mindestens einen Bereich eines Antikörpers menschlichen Ursprungs umfasst, und eine Bindungsspezifität für den α4β7-Komplex aufweist, und wobei der Antigen bindende Bereich drei Komplementaritäts bestimmende Bereiche (CDR1, CDR2 und CDR3) eines variablen Bereichs einer leichten Kette und drei Komplementaritäts bestimmende Bereiche (CDR1, CDR2 und CDR3) eines variablen Bereichs einer schweren Kette der nachfolgenden Aminosäuresequenzen umfasst:
| | |
|---|---|
| Leichte Kette: | CDR1 SEQ.ID.Nr. 9 |
| | CDR2 SEQ.ID.Nr. 10 |
| | CDR3 SEQ.ID.Nr. 11 |
| Schwere Kette: | CDR1 SEQ.ID.Nr. 12 |
| | CDR2 SEQ.ID.Nr. 13 |
| | CDR3 SEQ.ID.Nr. 14. |

2. Verwendung nach Anspruch 1, wobei das humanisierte Immunglobulin oder Antigen bindende Fragment davon einen variablen Bereich der schweren Kette der SEQ.ID.Nr. 6 umfasst.

3. Verwendung nach Anspruch 1 oder 2, wobei das humanisierte Immunglobulin oder Antigen bindende Fragment davon einen variablen Bereich der leichten Kette der SEQ.ID.Nr. 8 umfasst.

4. Verwendung nach Anspruch 1, wobei das Medikament ausreicht, um a) eine Sättigung der α4β7 Integrin-Bindungsstellen auf zirkulierenden Lymphozyten von 50 % oder darüber und/oder b) eine Inhibierung der a4β7 Integrin-Expression auf der Zelloberfläche zirkulierender Lymphozyten von 50 % oder darüber zu erhalten oder aufrecht zu halten, und wobei die Sättigung und/oder Inhibierung für eine Zeitspanne von mindestens 10 Tagen nach Verabreichung der Dosis aufrecht erhalten wird.

5. Verwendung nach Anspruch 4, wobei jede der Dosen unabhängig eine Menge an humanisiertem Immunglobulin oder Antigen-bindendem Fragment umfasst, die ausreichend ist um a) eine Sättigung der α4β7 Integrin-Bindungsstellen auf zirkulierenden Lymphozyten von 60 % oder darüber und/oder b) eine Inhibierung der α4β7 Integrin-Expression auf der Zelloberfläche zirkulierender Lymphozyten von 60 % oder darüber zu erhalten oder aufrecht zu halten.

6. Verwendung nach Anspruch 4, wobei jede der Dosen unabhängig eine Menge an humanisiertem Immunglobulin oder Antigen-bindendem Fragment umfasst, die ausreichend ist, um a) eine Sättigung der α4β7 Integrin-Bindungsstellen auf zirkulierenden Lymphozyten von 70 % oder darüber und/oder b) eine Inhibierung der α4β7 Integrin-Expression auf der Zelloberfläche zirkulierender Lymphozyten von 70 % oder darüber zu erhalten oder aufrecht zu halten.

7. Verwendung nach Anspruch 4, wobei jede der Dosen unabhängig eine Menge an humanisiertem Immunglobulin oder Antigen-bindendem Fragment umfasst, die ausreichend ist, um a) eine Sättigung der α4β7 Integrin-Bindungsstellen auf zirkulierenden Lymphozyten von 80 % oder darüber und/oder b) eine Inhibierung der α4β7 Integrin-Expression auf der Zelloberfläche zirkulierender Lymphozyten von 80 % oder darüber zu erhalten oder aufrecht zu halten.

8. Verwendung nach Anspruch 4, wobei jede der Dosen unabhängig eine Menge an humanisiertem Immunglobulin oder Antigen-bindendem Fragment umfasst, die ausreichend ist, um die Sättigung und/oder Inhibierung für eine Zeitspanne von mindestens etwa 14 Tagen, oder mindestens etwa 20 Tagen, oder mindestens etwa 25 Tagen, oder mindestens etwa 30 Tagen, oder mindestens etwa 60 Tagen nach Verabreichung der Dosis zu erhalten und aufrecht zu halten.

9. Verwendung nach Anspruch 1, wobei jede der Dosen unabhängig eine Menge an humanisiertem Immunglobulin oder Antigen-bindendem Fragment umfasst, die ausreichend ist, um eine Serum-Konzentration an humanem Immunglobulin von mindestens etwa 1 µg/ml für eine Zeitspanne von mindestens etwa 10 Tagen, oder mindestens etwa 14 Tagen, oder mindestens etwa 20 Tagen, oder mindestens etwa 25 Tagen, oder mindestens etwa 30 Tagen, oder mindestens etwa 60 Tagen nach Verabreichung der Dosis zu erhalten und aufrecht zu halten.

10. Verwendung nach einem der vorhergehenden Ansprüche, wobei jede der Dosen unabhängig etwa 0,1 bis etwa 8 mg humanisiertes Immunglobulin oder Antigen bindendes Fragment pro Kg Körpergewicht umfasst.

11. Verwendung nach einem der vorhergehenden Ansprüche, wobei jede der Dosen unabhängig etwa 0,1 bis etwa 5 mg humanisiertes Immunglobulin oder Antigen bindendes Fragment pro Kg Körpergewicht umfasst.

12. Verwendung nach einem der vorhergehenden Ansprüche, wobei jede der Dosen unabhängig etwa 0,1 bis etwa 2,5 mg humanisiertes Immunglobulin oder Antigen bindendes Fragment pro Kg Körpergewicht umfasst.

13. Verwendung nach einem der vorhergehenden Ansprüche, wobei jede der Dosen unabhängig etwa 0,15, etwa 0,5, etwa 1,0, etwa 1,5 oder etwa 2,0 mg humanisiertes Immunglobulin oder Antigen bindendes Fragment pro Kg Körpergewicht umfasst.

14. Verwendung nach einem der vorhergehenden Ansprüche, wobei das Intervall zwischen der anfänglichen Dosis und nachfolgenden Dosen mindestens etwa 14 Tage, oder mindestens etwa 21 Tage, oder mindestens etwa 28 Tage oder mindestens etwa 30 Tage beträgt.

15. Verwendung nach einem der vorhergehenden Ansprüche, welche weiter die Verwendung einer wirksamen Menge eines oder mehrerer therapeutischer Mittel bei der Herstellung des Medikaments umfasst.

16. Verwendung nach Anspruch 15, wobei die Mittel ausgewählt sind aus der Gruppe bestehend aus Steroiden, immunsupprimierenden Mitteln, nicht-steroidalen anti-entzündlichen Mitteln und Immunmodulatoren.

17. Verwendung nach Anspruch 1, wobei die Mittel ausgewählt sind aus der Gruppe bestehend aus Azathiopren, 6-Mercaptopurin, Sulfasalazin, 5-Aminosalicylsäure, Prednison und Prednisolon.

18. Verwendung nach einem der vorhergehenden Ansprüche, wobei die entzündliche Darmerkrankung eiternde Colitis ist.

19. Verwendung nach Anspruch 18, wobei die entzündliche Darmerkrankung Crohn's Erkrankung ist.

20. Verwendung nach Anspruch 1, wobei das minimale Intervall zwischen jeweils 2 Dosen eine Zeitspanne von mindestens 1 Woche ist.

21. Verwendung eines humanisierten Immunglobulins oder Antigen bindenden Fragments davon zur Herstellung eines Medikaments zur Verhinderung eines Rückfalls und/oder eines erneuten Wiederauftretens einer ruhenden entzündlichen Darmerkrankung, wobei das Medikament zur Verabreichung in einer einzigen Dosis oder einer anfänglichen Dosis gefolgt von einer oder mehreren Dosen gedacht ist, wobei während einer Zeitspanne von etwa 30 Tagen nicht mehr als 8 mg Immunglobulin oder Fragment pro Kg Körpergewicht verabreicht werden, und wobei das humanisierte Immunglobulin oder Antigen bindende Fragment davon einen Antigen bindenden Bereich nicht-humanen Ursprungs und mindestens einen Bereich eines Antikörpers menschlichen Ursprungs umfaßt, und eine Bindungsspezifität für den α4β7 Komplex aufweist, und wobei der Antigen bindende Bereich drei Komplementaritäts-bestimmende Bereiche (CDR1, CDR2 und CDR3) eines variablen Bereichs einer leichten Kette und drei Komplementaritäts-bestimmende Bereiche (CDR1, CDR2 und CDR3) eines variablen Bereichs einer schweren Kette der nachfolgenden Aminosäuresequenzen umfasst:
| | |
|---|---|
| Leichte Kette: | CDR1 SEQ.ID.Nr. 9 |
| | CDR2 SEQ.ID.Nr. 10 |
| | CDR3 SEQ.ID.Nr. 11 |
| Schwere Kette: | CDR1 SEQ.ID.Nr. 12 |
| | CDR2 SEQ.ID.Nr.13 |
| | CDR3 SEQ.ID.Nr. 14. |

22. Verwendung nach Anspruch 21, wobei der Ruhezustand durch medizinische oder chirurgische Therapie induziert wurde oder wobei die Darmerkrankung eiternde Colitis oder Crohn's Erkrankung ist.

23. Verwendung nach Anspruch 1 oder 4, wobei jede der Dosen unabhängig etwa 6 mg Immunglobulin oder ein Fragment davon pro Kg Körpergewicht umfasst.

24. Verwendung nach Anspruch 1 oder 4, wobei das Intervall zwischen den Dosen mindestens etwa 50 Tage beträgt.

## Revendications

1. Utilisation d'une immunoglobuline humanisée ou d'un fragment de celle-ci se liant à un antigène, pour la fabrication d'un médicament destiné au traitement d'un être humain présentant une affection abdominale inflammatoire, dans laquelle ledit médicament est destiné à être administré en une seule dose ou en une dose initiale suivie par une ou plusieurs doses ultérieures, dans laquelle pas plus d'environ 8 mg d'immunoglobuline ou de fragment par kg de poids corporel sont administrés pendant une période d'environ un mois, et
dans laquelle ladite immunoglobuline humanisée ou ledit fragment de celle-ci se liant à un antigène comprend une région se liant à un antigène d'origine non humaine et au moins une partie d'un anticorps d'origine humaine et possède une spécificité de liaison envers le complexe α4β7, et dans laquelle ladite région se liant à un antigène comprend trois régions de détermination de la complémentarité (CDR1, CDR2 et CDR3) d'une région variable de chaîne légère et trois régions de détermination de la complémentarité (CDR1, CDR2 et CDR3) d'une région variable de chaîne lourde de la séquence d'acides aminés exposée ci-dessous :
chaîne légère : CDR1 SEQ ID NO : 9
CDR2 SEQ ID NO : 10
CDR3 SEQ ID NO : 11
chaîne lourde : CDR1 SEQ ID NO : 12
CDR2 SEQ ID NO : 13
CDR3 SEQ ID NO : 14.

2. Utilisation selon la revendication 1, dans laquelle ladite immunoglobuline humanisée ou ledit fragment de celle-ci se liant à un antigène comprend la région variable de chaîne lourde de SEQ ID NO : 6.

3. Utilisation selon la revendication 1 ou 2, dans laquelle ladite immunoglobuline humanisée ou ledit fragment de celle-ci se liant à un antigène comprend la région variable de chaîne légère de SEQ ID NO : 8.

4. Utilisation selon la revendication 1, dans laquelle ledit médicament est suffisant pour atteindre et maintenir a) une saturation d'environ 50 % ou plus des sites de liaison de l'intégrine α4β7 sur les lymphocytes circulants et/ou b) une inhibition d'environ 50 % ou plus de l'expression de l'intégrine α4β7 sur la surface cellulaire des lymphocytes circulants, et dans laquelle ladite saturation et/ou ladite inhibition sont maintenues pendant une période d'au moins environ 10 jours après l'administration de ladite dose.

5. Utilisation selon la revendication 4, dans laquelle chacune desdites doses comprend indépendamment une quantité d'immunoglobuline humanisée ou de fragment se liant à un antigène qui est suffisante pour atteindre a) une saturation d'environ 60 % ou plus des sites de liaison de l'intégrine a4β7 sur les lymphocytes circulants et/ou b) une inhibition d'environ 60 % ou plus de l'expression de l'intégrine α4β7 sur la surface cellulaire des lymphocytes circulants.

6. Utilisation selon la revendication 4, dans laquelle chacune desdites doses comprend indépendamment une quantité d'immunoglobuline humanisée ou de fragment se liant à un antigène qui est suffisante pour atteindre a) une saturation d'environ 70 % ou plus des sites de liaison de l'intégrine α4β7 sur les lymphocytes circulants et/ou b) une inhibition d'environ 70 % ou plus de l'expression de l'intégrine α4β7 sur la surface cellulaire des lymphocytes circulants.

7. Utilisation selon la revendication 4, dans laquelle chacune desdites doses comprend indépendamment une quantité d'immunoglobuline humanisée ou de fragment se liant à un antigène qui est suffisante pour atteindre a) une saturation d'environ 80 % ou plus des sites de liaison de l'intégrine α4β7 sur les lymphocytes circulants et/ou b) une inhibition d'environ 80 % ou plus de l'expression de l'intégrine a4β7 sur la surface cellulaire des lymphocytes circulants.

8. Utilisation selon la revendication 4, dans laquelle chacune desdites doses comprend indépendamment une quantité d'immunoglobuline humanisée ou de fragment se liant à un antigène qui est suffisante pour atteindre et maintenir ladite saturation et/ou ladite inhibition pendant une période d'au moins environ 14 jours, ou d'au moins environ 20 jours, ou d'au moins environ 25 jours, ou d'au moins environ 30 jours, ou d'au moins environ 60 jours après l'administration de ladite dose.

9. Utilisation selon la revendication 1, dans laquelle chacune desdites doses comprend une quantité d'immunoglobuline humanisée qui est suffisante pour atteindre et maintenir une concentration sérique en immunoglobuline humanisée d'au moins environ 1 µg/mL pendant une période d'au moins environ 10 jours, ou d'au moins environ 14 jours, ou d'au moins environ 20 jours, ou d'au moins environ 25 jours, ou d'au moins environ 30 jours, ou d'au moins environ 60 jours après l'administration de ladite dose.

10. Utilisation selon l'une quelconque des revendications précédentes, dans laquelle chacune desdites doses comprend indépendamment environ 0,1 à environ 8 mg d'immunoglobuline humanisée ou de fragment se liant à un antigène par kg de poids corporel.

11. Utilisation selon l'une quelconque des revendications précédentes, dans laquelle chacune desdites doses comprend indépendamment d'environ 0,1 à environ 5 mg d'immunoglobuline humanisée ou de fragment se liant à un antigène par kg de poids corporel.

12. Utilisation selon l'une quelconque des revendications précédentes, dans laquelle chacune desdites doses comprend indépendamment environ 0,1 à environ 2,5 mg d'immunoglobuline humanisée ou de fragment se liant à un antigène par kg de poids corporel.

13. Utilisation selon l'une quelconque des revendications précédentes, dans laquelle chacune desdites doses comprend indépendamment environ 0,15, environ 0,5, environ 1,0, environ 1,5 ou environ 2,0 mg d'immunoglobuline humanisée ou de fragment se liant à un antigène par kg de poids corporel.

14. Utilisation selon l'une quelconque des revendications précédentes, dans laquelle l'intervalle entre la dose initiale et les doses subséquentes est d'au moins environ 14 jours, ou d'au moins environ 21 jours, ou d'au moins environ 28 jours, ou d'au moins environ 30 jours.

15. Utilisation selon l'une quelconque des revendications précédentes, comprenant en outre l'utilisation d'une quantité efficace d'un ou de plusieurs agents thérapeutiques supplémentaires dans la fabrication du médicament.

16. Utilisation selon la revendication 15, dans laquelle lesdits agents sont choisis dans le groupe constitué de stéroïdes, d'immunosuppresseurs, d'anti-inflammatoires non stéroïdiens et d'immunomodulateurs.

17. Utilisation selon la revendication 15, dans laquelle lesdits agents sont choisis dans le groupe constitué de l'azathioprène, de la 6-mercaptopurine, de la sulfasalazine, de l'acide 5-amino salicylique, de la prednisone et de la prednisolone.

18. Utilisation selon l'une quelconque des revendications précédentes, dans laquelle ladite affection abdominale inflammatoire est la rectocolite hémorragique.

19. Utilisation selon la revendication 18, dans laquelle ladite affection abdominale inflammatoire est la maladie de Crohn.

20. Utilisation selon la revendication 1, dans laquelle l'intervalle minimal entre deux doses est une période d'au moins environ 1 semaine.

21. Utilisation d'une immunoglobuline humanisée ou d'un fragment de celle-ci se liant à un antigène, pour la fabrication d'un médicament destiné à inhiber la rechute et/ou la récurrence d'une affection abdominale inflammatoire quiescente dans laquelle le médicament est destiné à être administré en une seule dose ou en une dose initiale et des doses subséquentes dans laquelle pas plus d'environ 8 mg d'immunoglobuline ou de fragment par kg de poids corporel par jour sont administrés durant une période d'environ 30 jours et dans laquelle ledit immunoglobuline humanisée ou ledit fragment d'immunoglobuline se liant à un antigène comprend un région se liant à un antigène d'origine non humaine et au moins une partie d'un anticorps d'origine humaine et possédant une spécificité de liaison pour le complexe α4β7, et dans laquelle ladite région se liant à un antigène comprend trois régions de détermination de la complémentarité (CDR1, CDR2 et CDR3) d'une région variable de chaîne légère et trois régions de détermination de la complémentarité (CDR1, CDR2 et CDR3) d'une région variable de chaîne lourde de la séquence d'acides aminés exposée ci-dessous :
chaîne légère : CDR1 SEQ ID NO : 9
CDR2 SEQ ID NO : 10
CDR3 SEQ ID NO : 11
chaîne lourde : CDR1 SEQ ID NO : 12
CDR2 SEQ ID NO : 13
CDR3 SEQ ID NO : 14.

22. Utilisation selon la revendication 21, dans laquelle la quiescence a été induite par une thérapie médicale ou chirurgicale ou dans laquelle ladite affection abdominale inflammatoire est la rectocolite hémorragique ou la maladie de Crohn.

23. Utilisation selon la revendication 1 ou 4, dans laquelle chacune des doses comprend indépendamment environ 6 mg d'immunoglobuline ou de fragment par kg de poids corporel.

24. Utilisation selon la revendication 1 ou 4, dans laquelle l'intervalle entre les doses est d'au moins environ 50 jours.
